# EUROPEAN PATENT SPECIFICATION

(11) **EP 3 281 014 B1**
(45) Date of publication and mention of the grant of the patent: **21.09.2022**
(21) Application number: 16716788.1
(22) Date of filing: 05.04.2016
(51) Int. Cl.: G01N 33/558, A61B 10/00, G01N 33/68, G01K 13/00

(54) **A TEST DEVICE FOR DETECTING AN ANALYTE IN A SALIVA SAMPLE AND METHOD OF USE**
TESTVORRICHTUNG ZUR DETEKTION EINES ANALYTS IN EINER SPEICHELPROBE UND VERFAHREN ZUR VERWENDUNG
DISPOSITIF D'ESSAI POUR DÉTECTION D'UNE SUBSTANCE À ANALYSER DANS UN ÉCHANTILLON DE SALIVE ET PROCÉDÉ D'UTILISATION

(30) Priority: 06.04.2015 US 201562143792 P
(43) Date of publication of application: 14.02.2018
(73) Proprietor: Bludiagnostics, Inc., Madison, WI 53703 (US)
(72) Inventor: BRENNER, Sarah, Katherine, Madison, WI 53705 (US); WEIBEL, Douglas, Benjamin, Bainbridge Island, WA 98110 (US)
(74) Representative: Witte, Weller & Partner Patentanwälte mbB
(86) International application number: PCT/US2016/026049
(87) International publication number: WO 2016/164365

(56) References cited:
- EP-A1- 2 465 469
- EP-A1- 2 465 469
- EP-A1- 2 465 469
- EP-A2- 1 051 616
- EP-A2- 1 051 616
- EP-A2- 1 301 790
- EP-A2- 1 301 790
- EP-A2- 1 301 790
- WO-A1-2008/106149
- WO-A1-2008/106149
- WO-A1-2008/106149
- WO-A1-2014/028687
- WO-A1-2014/028687
- WO-A1-2015/073878
- WO-A1-2015/073878
- CA-A1- 2 366 309
- CA-A1- 2 366 309
- US-A1- 2009 075 387
- US-A1- 2009 075 387
- US-A1- 2009 075 387
- US-A1- 2011 300 531
- US-A1- 2011 300 531
- US-A1- 2012 263 796
- US-A1- 2012 263 796
- US-A1- 2012 265 032
- US-A1- 2012 265 032
- US-A1- 2013 102 003
- US-A1- 2013 102 003
- US-A1- 2013 102 003
- US-A1- 2014 323 819
- US-A1- 2014 323 819
- US-B2- 7 763 433
- US-B2- 7 763 433
- MARTINA ZANGHERI ET AL: "A simple and compact smartphone accessory for quantitative chemiluminescence-based lateral flow immunoassay for salivary cortisol detection", BIOSENSORS AND BIOELECTRONICS, vol. 64, 1 February 2015 (2015-02-01), pages 63-68, XP055282181, AMSTERDAM, NL ISSN: 0956-5663, DOI: 10.1016/j.bios.2014.08.048
- JOU Y J ET AL: "Proteomic identification of salivary transferrin as a biomarker for early detection of oral cancer", ANALYTICA CHIMICA ACTA, ELSEVIER, AMSTERDAM, NL, vol. 681, no. 1-2, 29 November 2010 (2010-11-29), pages 41-48, XP027450898, ISSN: 0003-2670, DOI: 10.1016/J.ACA.2010.09.030 [retrieved on 2010-09-25]
- E B SCHWARTZ ET AL: "Transferrin Enzyme Immunoassay for Quantitative Monitoring of Blood Contamination in Saliva", CLINICAL CHEMISTRY, vol. 50, no. 3, 1 March 2004 (2004-03-01), pages 654-656, XP055282720, DOI: 10.1373/clinchem.2003.028266
- MARTINA ZANGHERI ET AL: "A simple and compact smartphone accessory for quantitative chemiluminescence-based lateral flow immunoassay for salivary cortisol detection", BIOSENSORS AND BIOELECTRONICS, vol. 64, 1 February 2015 (2015-02-01), pages 63-68, XP055282181, NL ISSN: 0956-5663, DOI: 10.1016/j.bios.2014.08.048
- JOU Y J ET AL: "Proteomic identification of salivary transferrin as a biomarker for early detection of oral cancer", ANALYTICA CHIMICA ACTA, ELSEVIER, AMSTERDAM, NL, vol. 681, no. 1-2, 29 November 2010 (2010-11-29), pages 41-48, XP027450898, ISSN: 0003-2670, DOI: 10.1016/J.ACA.2010.09.030 [retrieved on 2010-09-25]
- E B SCHWARTZ ET AL: "Transferrin Enzyme Immunoassay for Quantitative Monitoring of Blood Contamination in Saliva", CLINICAL CHEMISTRY, vol. 50, no. 3, 1 March 2004 (2004-03-01), pages 654-656, XP055282720,
- MARTINA ZANGHERI ET AL: "A simple and compact smartphone accessory for quantitative chemiluminescence-based lateral flow immunoassay for salivary cortisol detection", BIOSENSORS AND BIOELECTRONICS, vol. 64, 1 February 2015 (2015-02-01), pages 63-68, XP055282181, AMSTERDAM, NL ISSN: 0956-5663, DOI: 10.1016/j.bios.2014.08.048
- JOU Y J ET AL: "Proteomic identification of salivary transferrin as a biomarker for early detection of oral cancer", ANALYTICA CHIMICA ACTA, ELSEVIER, AMSTERDAM, NL, vol. 681, no. 1-2, 29 November 2010 (2010-11-29), pages 41-48, XP027450898, ISSN: 0003-2670, DOI: 10.1016/J.ACA.2010.09.030 [retrieved on 2010-09-25]
- E B SCHWARTZ ET AL: "Transferrin Enzyme Immunoassay for Quantitative Monitoring of Blood Contamination in Saliva", CLINICAL CHEMISTRY, vol. 50, no. 3, 1 March 2004 (2004-03-01), pages 654-656, XP055282720, DOI: 10.1373/clinchem.2003.028266

## Description

### FIELD

The present disclosure relates to devices, kits, instruments and methods for quantitatively detecting one or more analytes in a sample. More specifically, the present disclosure relates to a lateral flow test device, a kit or an instrument comprising the test device, and a method of using the test device, kit, or instrument for quantitatively detecting an analyte in a saliva sample, *e.g.,* a saliva sample from a subject, for example, for assessing hormone(s), ovulation, pregnancy, and/or fertility for a user, *e*.*g*., hormonal, ovulation, pregnancy, fertility status, time window, trend, or therapy monitoring or guidance for the user.

### BACKGROUND

In the following discussion, certain articles and methods are described for background and introductory purposes. Nothing contained herein is to be construed as an "admission" of prior art. Applicant expressly reserves the right to demonstrate, where appropriate, that the articles and methods referenced herein do not constitute prior art under the applicable statutory provisions.

Lateral flow immunoassays are widely used in many different areas of analytical chemistry and medicine, for example, in clinical diagnosis to determine the presence of an analyte of interest in a sample, such as a bodily fluid. Previous lateral flow immunoassay work is exemplified by U.S. patents and patent application publications: 5,602,040; 5,622,871; 5,656,503; 6,187,598; 6,228,660; 6,818,455; 2001/0008774; 2005/0244986; 6,352,862; 2003/0207465; 2003/0143755; 2003/0219908; 5,714,389; 5,989,921; 6,485,982; 11/035,047; 5,656,448; 5,559,041; 5,252,496; 5,728,587; 6,027,943; 6,506,612; 6,541,277; 6,737,277 B1; 5,073,484; 5,654,162; 6,020,147; 4,956,302; 5,120,643; 6,534,320; 4,942,522; 4,703,017; 4,743,560; 5,591,645; and RE 38,430 E. WO2008/106149A1 discloses a lateral flow assay for predicting the onset of menarche based on the detection of 17-beta estradiol, progesterone and/or further hormones in saliva. EP1051616, CA2366309, US2011300531 and WO2015073878A1 disclose the use of compounds such as creatinine and albumin as internal normalization substances in saliva.

Conventional lateral flow testing devices have typically required a relatively large sample volume and large amounts of conjugate. These devices usually require a blood, plasma, serum, or urine sample from a subject. In addition, they have also had a long wait time before the results of the test can be read.

Infertility is an age-old problem. The desire to bear children is one of the most fundamental biologically motivated human desires. Today, home-based pregnancy testing is qualitative and typically relies upon analyzing urine. These self-test pregnancy testing devices are typically used by women who suspect they may be pregnant. The device is usually a lateral flow immunoassay device, and normally the test is initiated by contacting a sampling portion of a lateral flow assay stick with a urine sample. The sampling portion of the assay stick may be immersed into a sample of urine in a container or, more typically, the user may urinate directly onto the sampling portion. The assay then runs without the woman needing to perform any further steps, and the result is indicated and read by eye or, in a digital device, is determined by an assay result reading means and displayed to the user, by means of a display such as, for example, a liquid crystal display (LCD). Conventional pregnancy tests work by measuring hCG (human chorionic gonadotrophin) in the urine sample. The hCG is produced by the developing embryo and a concentration of hCG in the sample above a certain threshold will trigger a positive result indicating pregnancy.

In the case of infertility, however, today's home-based testing offers very little insight into the period of fertility for end users. Women spend months trying to become pregnant, however the testing they perform each month tells them little about how their body is functioning and cannot assist physicians in making diagnoses.

There is a need for an accurate and easy-to-use technology for measuring fertility. The present disclosure addresses this and other related needs.

### BRIEF SUMMARY

In one aspect, disclosed herein is a lateral flow test device for quantitatively detecting an analyte in a saliva sample, *e.g.,* a saliva sample from a subject, according to claim 1, which device comprises: a first porous matrix that comprises two first test locations on said first porous matrix, one of said first test locations comprising a test reagent being able to compete with 17-beta estradiol in said sample for binding to a labeled Fab fragment that specifically binds to 17-beta estradiol, and the other said first test location comprising a test reagent being able to compete with progesterone in said sample for binding to a labeled Fab fragment that specifically binds to progesterone, wherein the saliva sample can flow laterally along said test device and can pass said two first test locations to form two first detectable signals, wherein said device further comprises a second porous matrix that comprises a second test location on said second porous matrix, said second test location comprising a second test reagent that is a normalization substance that is able to compete with a normalization substance in said saliva sample for binding to a binding reagent for said normalization substance, wherein the normalization substance is transferrin, albumin, creatinine, or a combination thereof, wherein a labeled Fab fragment that specifically binds to the normalization substance is dried on the test device, and wherein the saliva sample can flow laterally along said test device and pass said second test location to form a second detectable signal, and said two first detectable signals and said second detectable signal are able to be compared to assess levels of 17-beta estradiol and progesterone in said saliva sample, and said levels of 17-beta estradiol and progesterone in the saliva sample can be determined and can be combined into a single test result; said first porous matrix and said second porous matrix are the same porous matrix; and wherein the labeled Fab fragment that specifically binds to 17-beta estradiol, the labeled Fab fragment that specifically binds to progesterone, and the labeled fragment that specifically binds to the normalization substance, are each dried on the test device upstream from the first and second test locations.

In another aspect, the test device disclosed herein further comprises a temperature sensor that is configured to measure temperature of a subject while the device is inserted into the mouth of the subject.

In any of the preceding embodiments, the temperature sensor can comprise an electronic sleeve, a conductive ink, a temperature sensitive material, an optical measurement means, and/or an optical temperature sensor.

In any of the preceding embodiments, the temperature sensor can be comprised at least partially at a portion of the device configured to be inserted into the mouth of the subject.

According to the invention, the first porous matrix and the second porous matrix are the same matrix.

According to the invention, the second test reagent is a normalization substance that competes with a normalization substance in the saliva sample for binding to a binding reagent for the normalization substance, wherein the normalization substance is transferrin, albumin, creatinine, or a combination thereof.

In any of the preceding embodiments, the first test reagents and/or the second test reagent can be non-covalently bound to the first porous matrix and/or the second porous matrix. In one embodiment, the first test reagent and/or the second test reagent is or are linked to a carrier, *e.g.,* a carrier protein, to form a conjugate, and the conjugate(s) is or are immobilized to the first test site and/or the second test site on the first porous matrix and/or the second porous matrix.

In any of the preceding embodiments, the first test reagents and/or the second test reagent can be covalently bound to the first porous matrix and/or the second porous matrix. In one embodiment, the first test reagents and/or the second test reagent is or are covalently bound to the first test site and/or the second test site on the first porous matrix and/or the second porous matrix.

In any of the preceding embodiments, the first porous matrix and/or the second porous matrix can comprise nitrocellulose, glass fiber, polypropylene, polyethylene (preferably of very high molecular weight), polyvinylidene fluoride, ethylene vinylacetate, acrylonitrile and/or polytetrafluoro-ethylene.

In any of the preceding embodiments, the first porous matrix and/or the second porous matrix can be in the form a strip or a circle.

In any of the preceding embodiments, the first porous matrix and/or the second porous matrix can be a single element or can comprise multiple elements.

In any of the preceding embodiments, the test device disclosed herein can further comprise a sample application element(s) upstream from and in fluid communication with the matrix or matrices. In any of the preceding embodiments, the sample application element(s) can optionally comprise an absorbent material, *e*.*g*., a polymeric material and/or a cellulosic material. In one aspect, the sample application element is a saliva collector portion of the device. In one embodiment, the saliva collector portion is engineered to promote unidirectional flow of saliva toward another portion of the device, for example, a test region of the lateral flow assay device.

In any of the preceding embodiments, the test device disclosed herein can further comprise a liquid absorption element(s) downstream from and in fluid communication with the matrix or matrices.

In any of the preceding embodiments, at least a portion of the matrix or matrices can be supported by a solid backing.

In any of the preceding embodiments, a portion of the matrix or matrices, upstream from the test locations, can comprise a dried, labeled reagent, the labeled reagent capable of being moved by a liquid sample and/or a further liquid, *e.g.,* a sample transporting fluid or a washing fluid, to the first and/or second test location(s) and/or a positive and/or negative control location(s) to generate a detectable signal(s).

In any of the preceding embodiments, the dried, labeled reagent(s) can be located downstream from a sample application place(s) on the test device. In any of the preceding embodiments, the dried, labeled reagent(s) can be located upstream from a sample application place(s) on the test device.

In any of the preceding embodiments, the test device can further comprise, upstream from the test locations, a conjugate element(s) that comprises a dried, labeled reagent(s), the labeled reagent(s) being capable of being moved by a liquid sample and/or a further liquid to the test locations and/or a positive and/or negative control location to generate a detectable signal(s). In one embodiment, the conjugate element(s) is or are located downstream from a sample application place(s) on the test device. In another embodiment, the conjugate element(s) is or are located upstream from a sample application place(s) on the test device.

In any of the preceding embodiments, the label can be a soluble label, *e.g.,* a fluorescent label, or Tide Fluor 5.

In any of the preceding embodiments, the label can be a particle label, *e.g.,* a gold, latex particle label, Europium chelate (generally in latex or other microbeads) or Cellulose Nano Beads (manufactured by Asahi Kasei, Japan), wherein the particle label can comprise a covalently bound fluorophore or a fluorophore that is not covalently bound to the particle label *(e.g.,* a fluorophore that is trapped inside the particle label), and wherein the particle label optionally can be fluorescent (e.g., the particle label comprises a quantum dot).

In any of the preceding embodiments, the labeled reagent can be dried in the presence of a material that: a) stabilizes the labeled reagent; b) facilitates solubilization or resuspension of the labeled reagent in a liquid; c) facilitates mobility of the labeled reagent; and/or prevents aggregation of the labeled reagent (*e.g.*, the labeled reagent comprising a particle label). In one embodiment, the material is selected from the group consisting of: a protein, *e.g.*, a casein or BSA; a peptide; a polysaccharide; a sugar; a polymer, *e*.*g*., polyvinylpyrrolidone (PVP-40); a gelatin; a detergent, *e.g.*, Tween-20; a polyol, *e.g.*, mannitol; and combinations thereof (*e.g.*, a combination of at least two, at least three, at least four, or more of the listed materials).

In any of the preceding embodiments, the test device can further comprise: a control location comprising means for indicating proper flow of the liquid sample, indicating that the labeled reagent is added to the device, indicating that the labeled reagent is properly solubilized or dispersed, indicating a valid test result, indicating non-specific or unintended specific binding, or indicating heterophilic antibody interference, *e*.*g*., human anti-mouse antibody (HAMA) interference; and/or a reducing agent, for example, a reducing agent in the first and/or second porous matrix, *e*.*g*., for reducing a mucin in the sample, linearizing the structure of a mucin in the sample, and/or reducing sample viscosity to enable sample flow through the first and/or second porous matrix and laterally along the test device.

In any of the preceding embodiments, the test device can be configured for a saliva sample alone to transport the analyte and/or the labeled reagent to the test locations.

In any of the preceding embodiments, the test device can be configured for a developing liquid to be used to transport the analytes and/or the labeled reagent to the test locations.

In any of the preceding embodiments, the test device can further comprise a housing that covers at least a portion of the test device. In one embodiment, the housing comprises a sample application port to allow sample application upstream from or to the test location and/or an optic opening around the test locations to allow signal detection at the test location.

In any of the preceding embodiments, the housing can cover the entire test device.

In any of the preceding embodiments, at least a portion of the sample receiving portion of the matrix or the sample application element can be not covered by the housing and a sample or a buffer diluent can be applied to the portion of the sample receiving portion of the matrix or the sample application element outside the housing and then transported to the test location.

In any of the preceding embodiments, the housing can comprise a plastic material, laminated material, metal, glass, fiberglass, and/or ceramic.

In any of the preceding embodiments, the housing can comprise at least a portion of the temperature sensor that is configured to measure temperature of a subject while the device is inserted into the mouth of the subject.

In any of the preceding embodiments, the test device herein can further comprise a reader for detecting the first detectable signal, the second detectable signal and/or the temperature signal measured by the temperature sensor. In one embodiment, the reader is comprised in, on or within the housing.

In any of the preceding embodiments, the reader can be an optical reader, an electronic reader, a magnetic reader, or an electrochemical reader, or a combination thereof.

In any of the preceding embodiments, the porous matrix or the matrices and the housing comprising the temperature sensor and the reader can be configured to be releaseably assembled. In one embodiment, the assembly of the porous matrix or the matrices and the housing aligns the reader spatially within the device for detecting the first detectable signal, the second detectable signal and/or the temperature signal measured by the temperature sensor.

In any of the preceding embodiments, the porous matrix or the matrices can be configured to be a disposable unit.

In any of the preceding embodiments, the housing comprising the temperature sensor and the reader can be configured to be a reusable unit.

In any of the preceding embodiments, the test device can further comprise a liquid container.

In any of the preceding embodiments, the test device can further comprise machine-readable information, *e*.*g*., a barcode. In one embodiment, the barcode comprises lot specific information of the test device, *e.g.*, lot number of the test device. In another embodiment, the machine-readable information is comprised in a storage medium, *e*.*g*., a RFID device. In yet another embodiment, the RFID device comprises lot specific information, information on a liquid control or information to be used for quality control purpose.

In any of the preceding embodiments, the test device can further comprise a means for transmitting the signal(s) detected by the reader and/or the machine-readable information to another device. In one embodiment, the test device is configured for transmitting the signal(s) detected by the reader and/or the machine-readable information to another device via a wired connection between the test device and the other device. In another aspect, the test device is configured for transmitting the signal(s) detected by the reader and/or the machine-readable information to another device wirelessly.

In any of the preceding embodiments, the other device can be mobile phone, a tablet, a computer, an analytic device or system, a database, a LCD-enabled electronic monitor, or a combination thereof.

In any of the preceding embodiments, the second test location can comprise a second test reagent for quantitatively detecting transferrin, albumin, and/or creatinine as normalization substance(s).

In any of the preceding embodiments, the first detectable signals and the second detectable signal can be configured to be compared in a form of a ratio, an addition, a subtraction, a multiplication, and/or a division, or a combination thereof, to assess the amount of the analyte in the saliva sample, wherein a weighing factor is optionally applied to the first detectable signal or the second detectable signal or both before the comparison. According to the invention, the first detectable signal(s) for estradiol and/or progesterone and the second detectable signal(s) are configured to be compared to assess amount of estradiol and/or progesterone in the saliva sample. In one embodiment, the first detectable signal(s) for estradiol and/or progesterone and the second detectable signal(s) are configured to be compared in a form of a ratio, an addition, a subtraction, a multiplication, and/or a division, or a combination thereof, to assess amount of estradiol and/or progesterone in the saliva sample, wherein a weighing factor is optionally applied to the first detectable signal(s) or the second detectable signal(s) or both before the comparison.

In any of the preceding embodiments, an optical test can be used. In one embodiment, the total fluorescence on the assay sample line is determined, and a measurement of "background" fluorescence from a region of the assay that shouldn't have fluorescence is subtracted from the total fluorescence. This gives the fluorescence on the assay sample line. In another embodiment, the total fluorescence on the control sample line is determined, and a measurement of "background" fluorescence from a region of the assay that shouldn't have fluorescence is subtracted from the total fluorescence. This gives the fluorescence on the control sample line. Then, the fluorescence on the assay sample line and the fluorescence on the control sample line are compared to assess the amount of the analyte in the saliva sample. A weighting factor can be applied to the fluorescence on the assay sample line and/or the fluorescence on the control sample line before comparing the values.

In any of the preceding embodiments, the first detectable signal and the second detectable signal can be configured to be compared using a mathematical formula, an algorithm, a software and/or a computer.

According to the invention, the levels of estradiol and progesterone in a saliva sample can be determined and combined into a single test result.

In any of the preceding embodiments, the levels of the multiple analytes in a saliva sample can be combined into a single test result using a mathematical formula, an algorithm, a software and/or a computer.

In an embodiment, the levels of estradiol and progesterone in a saliva sample and temperature of a subject are determined and combined into a single test result.

In any of the preceding embodiments, the levels of the multiple analytes in the saliva sample and temperature of a subject can be combined into a single test result using a mathematical formula, an algorithm, software and/or a computer. In one embodiment, the level of estradiol and progesterone in a saliva sample and temperature of a subject are combined into a single test result using a mathematical formula, an algorithm, a software and/or a computer.

In one embodiment, the test device is configured for quantitatively detecting estradiol in a saliva sample from a subject ranging from about 1 pg/ml to about 30 pg/ml.

In any of the preceding embodiments, the test device can be configured for quantitatively detecting progesterone in a saliva sample from a subject ranging from about 50 pg/ml to about 500 pg/ml.

In any of the preceding embodiments, the test device can be configured for assessing hormone(s), ovulation, pregnancy, fertility, *e*.*g*., hormonal, ovulation, pregnancy, fertility status, time window, trend, or therapy monitoring or guidance. In one embodiment, the test device is configured for predicting ovulation. In another embodiment, the test device is configured for confirming pregnancy. In one embodiment, the test device is configured for assessing overall fertility and/or ability to conceive.

In any of the preceding embodiments, a liquid can have moved laterally along the test device to generate a detectable signal at the test location(s).

In another aspect, disclosed herein is a method for quantitatively detecting 17-beta estradiol and progesterone in a saliva sample, *e.g.,* a saliva sample from a subject, according to the attached claims, which method comprises: a) contacting a saliva sample with the test device of any of the preceding embodiments, wherein the saliva sample is applied to a site of the test device upstream of the first and second test locations, wherein the site optionally comprises an absorbent material, *e*.*g*., a polymeric material and/or a cellulosic material; b) transporting 17-beta estradiol and progesterone, if present in the saliva sample, and the labeled Fab fragment that specifically binds to 17-beta estradiol and the labeled Fab fragment that specifically binds to progesterone to the two first test location(s) and transporting the Fab fragment that specifically binds to the normalization substance to the second test location; and c) assessing the two first detectable signal(s) at the two first test locations, wherein: 1) the second detectable signal(s) at the second test location is assessed and compared to the first detectable signal(s) to assess levels of 17-beta estradiol and progesterone in said saliva sample; and 2) combining the levels of 17-beta estradiol and progesterone in the saliva sample into a single test result. In an embodiment, said method further comprises measuring temperature of a subject while the device is inserted into the mouth of the subject. In one aspect, the site of the test device upstream of the test location(s) is a saliva collector site of the device. In one embodiment, the saliva collector site is engineered to promote unidirectional flow of saliva toward another site of the device, for example, a test location on the device.

In any of the preceding embodiments, the liquid sample and the labeled reagent can be premixed to form a mixture and the mixture is applied to the test device. In one embodiment, the method further comprises a washing step after the mixture is applied to the test device. In another embodiment, the washing step comprises adding a washing liquid after the mixture is applied to the test device. In yet another embodiment, the test device comprises a liquid container comprising a washing liquid and the washing step comprises releasing the washing liquid from the liquid container.

In any of the preceding embodiments of the method, the test device can comprise a dried labeled reagent before use and the dried labeled reagent is solubilized or resuspended, and transported to the test location(s) by the liquid sample. In one embodiment, the dried labeled reagent is located downstream from the sample application site, and the dried labeled reagent is solubilized or resuspended, and transported to the test location by the liquid sample. In another embodiment, the dried labeled reagent is located upstream from the sample application site, and the dried labeled reagent is solubilized or resuspended, and transported to the test location by another liquid. In yet another embodiment, the labeled reagent is solubilized or resuspended, and transported to the test location by the liquid or saliva sample alone. In still another embodiment, the analyte and/or labeled reagent are solubilized or resuspended, and transported to the test location by another liquid.

In any of the preceding embodiments, the detectable signal can be assessed by a reader, such as an optical reader, an electronic reader, a magnetic reader, or an electrochemical reader, or a combination thereof. In one embodiment, the detectable signal is a fluorescent signal and the fluorescent signal is assessed by a fluorescent reader. In another embodiment, the fluorescent reader is a laser based or a light emitting diode (LED) based fluorescent reader.

In any of the preceding embodiments, the reader can comprise a single or multiple photodiodes or photodetectors, a charge-coupled device (CCD), a complementary metal-oxide semiconductor (CMOS), a camera *(e.g.,* a digital camera), or any combination thereof.

In any of the preceding embodiments, the method can further comprise measuring temperature of a subject while the device is inserted into the mouth of the subject.

In any of the preceding embodiments, the method can further comprise transmitting the signal(s) detected by the reader and/or the machine-readable information of the device to another device. In one embodiment, the method comprises transmitting the signal(s) detected by the reader and/or the machine-readable information to another device via a wired connection between the test device and the other device. In another embodiment, the method comprises transmitting the signal(s) detected by the reader and/or the machine-readable information to another device wirelessly.

In any of the preceding embodiments, the other device can be a mobile phone, a tablet, a computer, an analytic device or system, a database, a LCD-enabled electronic monitor, or a combination thereof.

In any of the preceding embodiments, the method can comprise quantitatively detecting an analyte in a saliva sample that is selected from the group consisting of cortisol, dehydroepiandrosterone (DHEA), dehydroepiandrosterone sulfate (DHEA-S), estriol, estradiol, estrone, progesterone, testosterone, thyroxine, triiodothyronine, thyroid stimulating hormone, androstenedione, alpha-amylase, C-reactive protein, melatonin, uric acid, interleukin 1-beta, interleukin-6, secretory immunoglobulin A, and a combination thereof. According to the invention, the method comprises quantitatively detecting estradiol and progesterone.

In any of the preceding embodiments, the method can comprise quantitatively detecting transferrin, albumin, creatinine, or a combination thereof, as a normalization substance.

In any of the preceding embodiments, the method can comprise comparing the two first detectable signals and the second detectable signal in a form of a ratio, an addition, a subtraction, a multiplication, and/or a division, or a combination thereof, to assess amount of the analytes in the saliva sample, wherein a weighing factor is optionally applied to the first detectable signal or the second detectable signal or both before the comparison. In one embodiment, the method comprises comparing the two first detectable signals for estradiol and progesterone and the second detectable signal(s) for transferrin and/or albumin to assess amount of estradiol and progesterone in the saliva sample. In another embodiment, the two first detectable signals for estradiol and progesterone and the second detectable signal(s) are configured to be compared in a form of a ratio, an addition, a subtraction, a multiplication, and/or a division, or a combination thereof, to assess amount of estradiol and progesterone in the saliva sample, wherein a weighing factor is optionally applied to the two first detectable signals or the second detectable signal(s) or both before the comparison.

In any of the preceding embodiments of the method, the two first detectable signal and the second detectable signal are compared using a mathematical formula, an algorithm, a software and/or a computer.

According to the invention, the levels of estradiol and progesterone in a saliva sample are determined and combined into a single test result.

In any of the preceding embodiments, the levels of the multiple analytes in a saliva sample can be combined into a single test result using a mathematical formula, an algorithm, a software and/or a computer.

In an embodiment, levels of estradiol and progesterone in a saliva sample and temperature of a subject are determined and combined into a single test result.

In one embodiment, the level of estradiol and progesterone in the saliva sample and temperature of a subject are combined into a single test result using a mathematical formula, an algorithm, a software and/or a computer.

In any of the preceding embodiments, the method can be used for quantitatively detecting estradiol in a saliva sample from a subject ranging from about 1 pg/ml to about 30 pg/ml. In one embodiment, the method is used for quantitatively detecting progesterone in a saliva sample from a subject ranging from about 50 pg/ml to about 500 pg/ml.

In any of the preceding embodiments, the method is used for assessing hormone(s), ovulation, pregnancy, fertility, *e*.*g*., hormonal, ovulation, pregnancy, fertility status, time window, trend, or therapy monitoring or guidance. In one embodiment, the method is used for predicting ovulation. In one embodiment, the method is used for confirming pregnancy. In one embodiment, the method is used for assessing overall fertility and/or ability to conceive, for family planning, and/or for birth control.

In any of the preceding embodiments of the test device or method, the subject can be a mammal. In one embodiment, the mammal is a human. In another embodiment, the mammal is a non-human mammal.

In any of the preceding embodiments of the test device or method, the subject can be a farm animal, an economical animal or a pet, *e.g.,* a bovine, equine, or canine.

Disclosed herein is The invention can be used in a kit for quantitatively detecting the analytes in a saliva sample, *e.g.*, a saliva sample from a subject, which kit comprises: a) a test device of any of the preceding embodiments; and b) an instruction for using the test device for quantitatively detecting the analytes in a saliva sample.

### BRIEF DESCRIPTION OF THE DRAWINGS

**FIG. 1** illustrates a lateral flow assay test strip embedded within a cartridge, according to one aspect of the present disclosure.
**FIGS. 2A-2C** illustrates an electronic reader for the lateral flow test device, according to one aspect of the present disclosure. **FIG. 2A** shows the reader. **FIG. 2B** shows a top view of the test strip inserting into the reader. **FIG. 2C** shows a side view of the test strip inserting into the reader.
**FIG. 3** illustrates an electronic reader for the lateral flow test device, according to one aspect of the present disclosure.
**FIG. 4** is a flowchart showing a method of using the lateral flow test device, according to one aspect of the present disclosure.
**FIG. 5** depicts a general schematic diagram of the components of a hormone monitoring system, according to one aspect of the present disclosure.

### DETAILED DESCRIPTION

### A. Definitions

Unless defined otherwise, all technical and scientific terms used herein have the same meaning as is commonly understood by one of ordinary skill in the art to which the present disclosure belongs. If a definition set forth in this section is contrary to or otherwise inconsistent with a definition set forth in the patents, applications, published applications and other publications that mentioned herein, the definition set forth in this section prevails.

A plurality of hardware and software based devices, as well as a plurality of different structural components may be used to implement the present disclosure. In addition, it should be understood that embodiments of the present disclosure may include hardware, software, and electronic components or modules that, for purposes of discussion, may be illustrated and described as if the majority of the components were implemented solely in hardware. However, one of ordinary skill in the art, and based on a reading of this detailed description, would recognize that, in at least one embodiment, the electronic based aspects of the present disclosure may be implemented in software (*e*.*g*., stored on non-transitory computer-readable medium) executable by one or more processors. As such, it should be noted that a plurality of hardware and software based devices, as well as a plurality of different structural components may be utilized to implement the present disclosure.

The use of "including," "comprising," or "having" and variations thereof herein is meant to encompass the items listed thereafter and equivalents thereof as well as additional items. The use of any and all examples, or exemplary language (*e*.*g*., "such as") is intended to better illuminate the embodiments and does not pose a limitation on the scope of the claims unless otherwise stated.

As used herein and in the appended claims, the singular forms "a," "an," and "the" include plural referents unless the context clearly dictates otherwise. Similarly, when the plural form is used it is to be construed to cover the singular form as the context permits. For example, "a" or "an" means "at least one" or "one or more." Thus, reference to "an analyte" refers to one or more analytes, and reference to "the method" includes reference to equivalent steps and methods disclosed herein and/or known to those skilled in the art, and so forth.

Throughout this disclosure, various aspects of the claimed subject matter are presented in a range format. It should be understood that the description in range format is merely for convenience and brevity and should not be construed as an inflexible limitation on the scope of the claimed subject matter. Accordingly, the description of a range should be considered to have specifically disclosed all the possible sub-ranges as well as individual numerical values within that range. For example, where a range of values is provided, it is understood that each intervening value, between the upper and lower limit of that range and any other stated or intervening value in that stated range is encompassed within the claimed subject matter. The upper and lower limits of these smaller ranges may independently be included in the smaller ranges, and are also encompassed within the claimed subject matter, subject to any specifically excluded limit in the stated range. Where the stated range includes one or both of the limits, ranges excluding either or both of those included limits are also included in the claimed subject matter. This applies regardless of the breadth of the range.

As used herein, an "individual" or a "subject" can be any living organism, including humans and other mammals. As used herein, the term "subject" is not limited to a specific species or sample type. For example, the term "subject" may refer to a patient, and frequently a human patient. However, this term is not limited to humans and thus encompasses a variety of mammalian or other species. In one embodiment, the subject can be a mammal or a cell, a tissue, an organ or a part of the mammal. Mammals include any of the mammalian class of species, preferably human (including humans, human subjects, or human patients). Mammals include, but are not limited to, farm animals, sport animals, pets, primates, horses, dogs, cats, mice and rats.

As used herein, the term "sample" refers to anything which may contain an analyte for which an analyte assay is desired. As used herein, a "biological sample" can refer to any sample obtained from a living or viral source or other source of macromolecules and biomolecules, and includes any cell type or tissue of a subject from which nucleic acid or protein or other macromolecule can be obtained. The biological sample can be a sample obtained directly from a biological source or a sample that is processed. For example, isolated nucleic acids that are amplified constitute a biological sample. Biological samples include, but are not limited to, body fluids, such as saliva, urine, blood, plasma, serum, semen, stool, sputum, cerebrospinal fluid, synovial fluid, sweat, tears, mucus, amniotic fluid, tissue and organ samples from animals and plants and processed samples derived therefrom. Examples of biological tissues also include organs, tumors, lymph nodes, arteries and individual cell(s).

As used herein, "quantitatively detecting an analyte or analytes" means that each of the analytes is determined with a precision, or coefficient of variation (CV), at about 30% or less, at analyte level(s) or concentration(s) that encompasses one or more desired threshold values of the analyte(s), and/or at analyte level(s) or concentration(s) that is below, at about low end, within, at about high end, and/or above one or more desired reference ranges of the analyte(s). In some embodiments, it is often desirable or important to have higher precision, *e.g.*, CV less than 30%, 25%, 20%, 15%, 10%, 9%, 8%, 7%, 6%, 5%, 4%, 3%, 2%, 1%, 0.5%, 0.1%, or smaller. In other embodiments, it is often desirable or important that the analytes are quantified with a desired or required CV at analyte level(s) or concentration(s) that is substantially lower than, at about, or at, and/or substantially higher than the desired or required threshold values of the analyte(s). In still other embodiments, it is often desirable or important that the analytes are quantified with a desired or required CV at analyte level(s) or concentration(s) that is substantially lower than the low end of the reference range(s), that encompasses at least 10%, 20%, 30%, 40%, 50%, 60%, 70%, 80%, 90%, 95%, or the entire reference range(s), and/or that is substantially higher than the high end of the reference range(s).

As used herein, an analyte level or concentration "at about" a threshold value or a particular point, *e.g.,* low or high end, of a reference range, means that the analyte level or concentration is at least within plus or minus 20% of the threshold value or the particular point, *e.g.,* low or high end, of the reference range. In other words, an analyte level or concentration "at about" a threshold value or a particular point of a reference range means that the analyte level or concentration is at from 80% to 120% of the threshold value or a particular point of the reference range. In some embodiments, an analyte level or concentration "at about" a threshold value or a particular point of a reference range means that the analyte level or concentration is at least within plus or minus 15%, 10%, 5%, 4%, 3%, 2%, 1%, or equals to the threshold value or the particular point of the reference range.

As used herein, analyte level or concentration that is "substantially lower than" a threshold value or the low end of a reference range means that the analyte level or concentration is at least within minus 50% of the threshold value or the low end of the reference range. In other words, an analyte level or concentration that is "substantially lower than" the threshold value or the low end of the reference range means that the analyte level or concentration is at least at 50% of the threshold value or the low end of the reference range. In some embodiments, analyte level or concentration that is "substantially lower than" the threshold value or the low end of the reference range means that the analyte level or concentration is at least at 60%, 70%, 80%, 90%, 95%, 96%, 97%, 98%, 99% of the threshold value or the low end of the reference range.

As used herein, analyte level or concentration that is "substantially higher than" a threshold value or the high end of a reference range means that the analyte level or concentration is at least within plus 5 folds of the threshold value or the high end of the reference range. In other words, an analyte level or concentration that is "substantially higher than" the threshold value or the high end of the reference range means that the analyte level or concentration is at 101% to 5 folds of the threshold value or the high end of the reference range. In some embodiments, analyte level or concentration that is "substantially higher than" the threshold value or the high end of the reference range means that the analyte level or concentration is at least at 101%, 102%, 103%, 104%, 105%, 110%, 120%, 130%, 140%, 150%, 2 folds, 3 folds, 4 folds or 5 folds of the threshold value or the high end of the reference range.

As used herein, "threshold value" refers to an analyte level or concentration obtained from samples of desired subjects or population (*e*.*g*., women who are pregnant or not pregnant, or women at a particular stage of pregnancy), *e*.*g*., values of analyte level or concentration found in normal, clinically healthy individuals, analyte level or concentration found in "diseased" subjects or population, or analyte level or concentration determined previously from samples of desired subjects or population. If a "normal value" is used as a "threshold range," depending on the particular test, a result can be considered abnormal if the value of the analyte level or concentration is more or less than the normal value. A "threshold value" can be based on calibrated or un-calibrated analyte levels or concentrations.

As used herein, "reference range" refers to a range of analyte level or concentration obtained from samples of a desired subjects or population (*e*.*g*., women who are pregnant or not pregnant, or women at a particular stage of pregnancy), *e*.*g*., the range of values of analyte level or concentration found in normal, clinically healthy individuals, the range of values of analyte level or concentration found in "diseased" subjects or population, or the range of values of analyte level or concentration determined previously from samples of desired subjects or population. If a "normal range" is used as a "reference range," a result is considered abnormal if the value of the analyte level or concentration is less than the lower limit of the normal range or is greater than the upper limit. A "reference range" can be based on calibrated or un calibrated analyte levels or concentrations.

As used herein, "antibody" refers a peptide or polypeptide derived from, modeled after or substantially encoded by an immunoglobulin gene or immunoglobulin genes, or fragments thereof, capable of specifically binding an antigen or epitope. *See, e.g.* Fundamental Immunology, 3rd Edition, W.E. Paul, ed., Raven Press, N.Y. (1993); Wilson (1994; J. Immunol. Methods 175:267-273; Yarmush (1992) J. Biochem. Biophys. Methods 25:85-97. The term antibody includes antigen-binding portions, *i.e.*, "antigen binding sites," (*e.g.*, fragments, subsequences, complementarity determining regions (CDRs)) that retain capacity to bind antigen, including (i) a Fab fragment, a monovalent fragment consisting of the VL, VH, CL and CHI domains; (ii) a F(ab')2 fragment, a bivalent fragment comprising two Fab fragments linked by a disulfide bridge at the hinge region; (iii) a Fd fragment consisting of the VH and CHI domains; (iv) a Fv fragment consisting of the VL and VH domains of a single arm of an antibody, (v) a dAb fragment (Ward et al., (1989) Nature 341:544-546), which consists of a VH domain; and (vi) an isolated complementarity determining region (CDR). Single chain antibodies are also included by reference in the term "antibody." An "antibody" may be naturally occurring or man-made such as monoclonal antibodies produced by conventional hybridoma technology, various display methods, *e*.*g*., phage display, and/or a functional fragment thereof.

The term "epitope" refers to an antigenic determinant capable of specific binding to an antibody. Epitopes usually or often consist of chemically active surface groupings of molecules such as amino acids or sugar side chains and can have specific three dimensional structural characteristics, as well as specific charge characteristics. Conformational and nonconformational epitopes are distinguished in that the binding to the former but not the latter is lost in the presence of denaturing solvents.

As used herein, "monoclonal antibody" refers to an antibody obtained from a population of substantially homogeneous antibodies, *i.e.*, the antibodies comprising the population are identical except for possible naturally occurring mutations that are present in minor amounts. As used herein, a "monoclonal antibody" further refers to functional fragments of monoclonal antibodies.

As used herein, a "binding reagent" refers to any substance that binds to a target or an analyte with desired affinity and/or specificity. Non-limiting examples of the binding reagent include cells, cellular organelles, viruses, particles, microparticles, molecules, or an aggregate or complex thereof, or an aggregate or complex of molecules. Exemplary binding reagents can be an amino acid, a peptide, a protein, *e.g.,* an antibody or receptor, a nucleoside, a nucleotide, an oligonucleotide, a nucleic acid, *e.g.,* DNA or RNA, a vitamin, a monosaccharide, an oligosaccharide, a carbohydrate, a lipid, an aptamer and a complex thereof.

As used herein, the term "specifically binds" refers to the specificity of a binding reagent, *e.g.,* an antibody or an aptamer, such that the binding reagent preferentially binds to a defined target or analyte. A binding reagent "specifically binds" to a target if it binds with greater affinity, avidity, more readily, and/or with greater duration than it binds to other substances. For example, a binding reagent that specifically binds to a target may bind to the target analyte with at least about 10%, at least about 20%, at least about 30%, at least about 40%, at least about 50%, at least about 60%, at least about 70%, at least about 80%, at least about 90% or more, greater affinity as compared to binding to other substances; or with at least about two-fold, at least about five-fold, at least about ten-fold or more of the affinity for binding to a target analyte as compared to its binding to other substances. Recognition by a binding reagent of a target analyte in the presence of other potential interfering substances is also one characteristic of specifically binding. Preferably, a binding reagent, *e.g.,* an antibody or an aptamer, that is specific for or binds specifically to a target analyte, avoids binding to a significant percentage of non-target substances, *e*.*g*., non-target substances present in a testing sample. In some embodiments, a binding reagent avoids binding greater than about 90% of non-target substances, although higher percentages are clearly contemplated and preferred. For example, a binding reagent can avoid binding about 91%, about 92%, about 93%, about 94%, about 95%, about 96%, about 97%, about 98%, about 99%, about 99% and about 99.9% or more of non-target substances. In other embodiments, a binding reagent can avoid binding greater than about 10%, 20%, 30%, 40%, 50%, 60%, or 70%, or greater than about 75%, or greater than about 80%, or greater than about 85% of non-target substances.

### B. Devices and kits for quantitatively detecting an analyte in a saliva sample

There are no commercially available, convenient tests for measuring levels of fertility-related hormones. Standard hormone tests are performed on samples of blood drawn in a doctor's office, however these tests do not distinguish between active and inactive estrogen and progesterone. Critically, active concentrations of these hormones are important indicators of fertility, as their concentration directly correlates with the function (e.g., ovulation) of female reproductive organs; concentrations of their inactive forms are not as closely correlated with ovulation and thereby complicate analyses. The ability to track active forms throughout the month in an accurate and immediate manner will provide unprecedented information not currently available to women trying to conceive. This capability will accelerate diagnosis and treatment for women experiencing infertility. One month of daily monitoring using this device may provide physicians with information they need to identify a potential hormonal imbalance associated with infertility. Additionally, the same device could be used to help in diagnosing and treating other hormonal imbalance disorders including estrogen and testosterone hormone therapies.

An accurate and easy-to-use technology for measuring fertility is desired. In one aspect, disclosed herein is a technology that delivers early answers, diagnosing pregnancy as early as ultrasound technology, an average of five days sooner than currently available home pregnancy tests. While trying to become pregnant, women obtain quantitative information about their body that is currently unavailable with any other test, including those most commonly administered at the doctor's office. The information from this new technology will enable physicians to make infertility-related diagnoses. In one aspect, the device and method disclosed herein requires only a small sample of saliva, making it possible for women to test discretely and comfortably.

The device of the invention can be used to measure the concentration of the mentioned hormones in bodily fluids, such as saliva, which comprises: (1) an assay device for collecting body fluids and determining hormone concentrations, (2) an electronic device for reading the results of the assay, and (3) a thermometer for measuring the temperature of the user. In some aspects, the results of said device are then transferred, wirelessly or through a cable, to a computerized device to process and display the information.

With the method of the invention, the hormone levels of a user can be monitored. In some embodiments, the method comprises collecting a saliva sample of a user and performing an assay on the saliva sample to determine a chemical concentration of the mentioned bioavailable hormones. In some embodiments, the method further comprises measuring a basal temperature of the user and analyzing the assay and the basal temperature to produce a quantified result. In some embodiments, the quantified result is transmitted to a software program on a computerized device, where the computerized device has a graphical user interface that displays the assay results. Other aspects of the invention will become apparent by consideration of the detailed description and accompanying drawings.

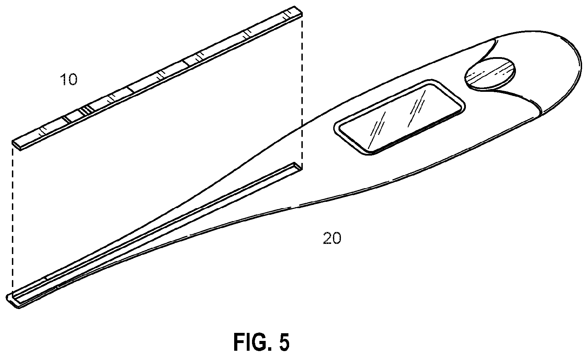

In one embodiment, a system and method of monitoring fertility is provided. For example, the system measures the active form of the mentioned hormones in saliva using an integrated assay and a basal temperature. In one embodiment, the system comprises at least three components. In one embodiment, the first component (*e*.*g*., component **10** in **FIG. 5**) of the system comprises a collecting device for collecting a saliva sample of a user. The collecting device is used to perform an assay on the saliva sample. According to the invention, the collection device is used to perform an assay on the saliva sample to determine the concentration of bioavailable progesterone and estradiol. The collecting device can comprise a disposable material such as paper or can comprise a reusable material such as plastic.

In one embodiment, the second component (e.g., component **20** in **FIG. 5**) of the system comprises a thin electronic sleeve that measures the basal temperature of the user. In one embodiment, the electronic sleeve analyzes the measured basal temperature and the result of the assay performed by the collecting device and quantifies the data. The quantified data can then be produced as a quantitative readout that the user can understand. The electronic sleeve connects to a smartphone or tablet (e.g., using a cable or by wireless communication) through which it delivers the quantitative readout to the user.

In one embodiment, the third component of the system comprises a software application on a smartphone or a tablet. The software application processes and displays the readout of the device and can share it with other electronic resources.

As an alternative to simultaneously measuring the active hormone and basal body temperature, another embodiment comprises independently measuring the basal body temperature and entering the ancillary information into the software for processing and transmission.

Disclosed herein is a device for monitoring the mentioned hormone levels of the user, the device comprising: a collection device for obtaining a bodily fluid sample from a user; an assay device to determine the active hormone concentrations in the sample; an electronic device that can interpret and quantitate a result of the assay, and a computerized device able to receive and process the information. In one embodiment, the device comprises a component for measuring a basal body temperature of the user.

Disclosed herein is a method of monitoring the mentioned hormone levels of a user, the method comprising: collecting a saliva sample of a user; performing an assay on the saliva sample to determine a chemical concentration of bioavailable hormones; analyzing the assay and the basal temperature and producing a quantified result; transmitting the quantified result to a software program on a computerized device, the computerized device having a user interface, and displaying the quantified result on the user interface in the form of a readout via the software program. In one embodiment, the method comprises an additional step of measuring a basal temperature of the user.

In yet another aspect, disclosed herein is a device designed to collect bodily fluids and quantitatively measure the mentioned hormones. In one embodiment, disclosed herein is a device comprising: (a) a collection component for bodily fluids, (b) a component for measuring a basal body temperature of an animal, (c) a component for measuring a result of an assay performed on the body fluid, and (d) a computerized device for analyzing an output of a reading and transferring the output to other electronic records.

While specific embodiments have been described, the invention described here should only be limited by the attached claims and their equivalents. Various features and advantages of the invention are set forth in the following claims. The principles of the present test devices, kits, systems and methods can be applied, or can be adapted to apply, to the lateral flow test devices and assays known in the art. For example, the principles of the present test devices, kits, systems and methods can be applied, or can be adapted to apply, to the lateral flow test devices and assays disclosed and/or claimed in the following patents and applications: 5,073,484, 5,654,162, 6,020,147, 4,695,554, 4,703,017, 4,743,560, 5,591,645, RE 38,430 E, 5,602,040, 5,633,871, 5,656,503, 6,187,598, 6,228,660, 6,818,455, 7,109,042, 6,352,862, 7,238,537, 7,384,796, 7,407,813, 5,714,389, 5,989,921, 6,485,982, 5,120,643, 5,578,577, 6,534,320, 4,956,302, RE 39,664 E, 5,252,496, 5,559,041, 5,728,587, 6,027,943, 6,506,612, 6,541,277, 6,737,277, 7,175,992 B2, 7,691,595 B2, 6,770,487 B2, 7,247,500 B2, 7,662,643 B2, 5,712,170, 5,965,458, 7,371,582 B2, 7,476,549 B2, 7,633,620 B2, 7,815,853 B2, 6,267,722 B1, 6,394,952 B1, 6,867,051 B1, 6,936,476 B1, 7,270,970 B2, 7,239,394 B2, 7,315,378 B2, 7,317,532 B2, 7,616,315 B2, 7,521,259 B2, 7,521,260 B2, US 2005/0221504 A1, US 2005/0221505 A1, US 2006/0240541 A1, US 2007/0143035 A1, US 2007/0185679 A1, US 2008/0028261 A1, US 2009/0180925 A1, US 2009/0180926 A1, US 2009/0180927 A1, US 2009/0180928 A1, US 2009/0180929 A1, US 2009/0214383 A1, US 2009/0269858A1, 6,777,198, US 2009/0311724 A1, US 2009/0117006 A1, 7,256,053, 6,916,666, 6,812,038, 5,710,005, 6,140,134, US 2010/0143941 A1, 6,140,048, 6,756,202, 7,205,553, 7,679,745, US 2010/0165338 A1, US 2010/0015611 A1, 5,422,726, 5,596,414, 7,178,416, 7,784,678 B2, US 2010/094564 A1, US 2010/0173423 A1, US 2009/0157023 A1, 7,785,899, 7,763,454 B2, US 2010/0239460 A1, US 2010/0240149 A1, 7,796,266 B2, 7,815,854 B2, US 2005/0244953 A1, US 2007/0121113 A1, US 2003/0119202 A1, US 2010/0311181 A1, 6,707,554 B1, 6,194,222 B1, 7,713,703, EP 0,149,168 A1, EP 0,323,605 A1, EP 0,250,137 A2, GB 1,526,708 and WO99/40438.

In one aspect, the present invention provides a lateral flow test device for quantitatively detecting 17-beta estradiol and progesterone in a saliva sample, *e.g.,* a saliva sample from a subject, which device comprises: a first porous matrix that comprises two first test locations on said first porous matrix, one of said first test locations comprising a test reagent being able to compete with 17-beta estradiol in said sample for binding to a labeled Fab fragment that specifically binds to 17-beta estradiol, and the other said first test location comprising a test reagent being able to compete with progesterone in said sample for binding to a labeled Fab fragment that specifically binds to progesterone, wherein the saliva sample flows laterally along said test device and passes said two first test location to form two first detectable signals, wherein: 1) said device further comprises a second porous matrix that comprises a second test location on said second porous matrix, said second test location comprising a second test reagent that is a normalization substance that is able to compete with a normalization substance in said saliva sample for binding to a binding reagent for said normalization substance, wherein the normalization substance is transferrin, albumin, creatinine, or a combination thereof, wherein a labeled Fab fragment that specifically binds to the normalization substance is dried on the test device, wherein the saliva sample can flow laterally along said test device and pass said second test location to form a second detectable signal, and said first detectable signals and said second detectable signal are configured to be compared to assess levels of said analyte in said saliva sample. In an embodiment, said device further comprises a temperature sensor that is configured to measure temperature of a subject while said device is inserted into the mouth of said subject.

The test device disclosed herein can further comprise a temperature sensor that is configured to measure temperature of a subject while the device is inserted into the mouth of the subject. The temperature sensor can comprise an electronic sleeve. In other embodiments, the temperature sensor can get integrated on any part of the lateral flow device, for example, on the membrane of the lateral flow device. A temperature sensor can be integrated on the device, for example, by applying a defined structure of conductive material resistances in a defined range. With changing temperature, the resistance of the printed electronic temperature sensor will change in a defined way, allowing the temperature to be measured.

In any of the preceding embodiments, the temperature sensor can be comprised at least partially at a portion of the device configured to be inserted into the mouth of the subject.

The sensor(s) for the lateral flow devices according to any of the embodiments can comprise an electronic sleeve for sensing the basal temperature of a subject, or can be prepared using printed electronics (*e*.*g*., conductive inks and temperature sensitive materials, such as conductive platinum ink (DuPont BQ321)). As discussed they can be printed either directly on (for example) the nitrocellulose, on the backing material, or on a top layer that is attached to the device. Likewise, in an alternative embodiment, non-printed electrodes may be applied either directly onto the absorbent material (such as the nitrocellulose membrane), or through the backing material of the device. The interface to the electronic integrated circuits can be made by use of flex circuits or similar technologies.

The matrix can comprise any suitable material(s). For example, the matrix can comprise nitrocellulose, glass fiber, polypropylene, polyethylene (preferably of very high molecular weight), polyvinylidene flouride, ethylene vinylacetate, acrylonitrile and/or polytetrafluoro-ethylene. In any of the preceding embodiments, the first porous matrix and the second porous matrix can be two distinct matrices. In any of the preceding embodiments, the first porous matrix and the second porous matrix can be the same matrix.

The matrix can have any suitable form. In some embodiments, the matrix can be in the form a strip or a circle. In other embodiments, the matrix can be a single element or can comprise multiple elements.

The test device can comprise additional elements. In some embodiments, the test device can further comprise a sample application element upstream from and in fluid communication with the matrix. In other embodiments, the test device can further comprise a liquid absorption element downstream from and in fluid communication with the matrix.

In some embodiments, at least a portion of the matrix is supported by a solid backing. In other embodiments, half, more than half or all portion of the matrix is supported by a solid backing. The solid backing can be made of any suitable material, *e*.*g*., solid plastics. If the test device comprises electrode or other electrical elements, the solid backing should generally comprise non-conductive materials.

The dried, labeled reagent can be located at any suitable locations. In some embodiments, the dried, labeled reagent is located downstream from a sample application place on the test device. In other embodiments, the dried, labeled reagent is located upstream from a sample application place on the test device. In still other embodiments, the test device further comprises, upstream from the test locations, a conjugate element that comprises a dried, labeled reagent, the labeled reagent being capable of being moved by a liquid sample and/or a further liquid to the test locations and/or a control location, *e.g.,* a positive and/or negative control location, to generate a detectable signal. The conjugate element can be located downstream from a sample application place on the test device. Alternatively, the conjugate element can be located upstream from a sample application place on the test device.

The labeled reagent can have any suitable binding affinity and/or specificity. In some embodiments, the labeled reagent binds, and preferably specifically binds, to one or more analytes in the sample. In other embodiments, the test device comprises multiple labeled reagents, wherein each of the labeled reagents competes with a different analyte in the sample for binding to a binding reagent for the analyte at a test location.

Any suitable label can be used depending on the intended detection methods. The label can be a direct label or an indirect label. A direct label can be detected by an instrument, device or naked eyes without further step to generate a detectable signal. A visual direct label, *e.g.,* a gold or latex particle label, can be detected by naked eyes. An indirect label, *e.g.,* an enzyme label, requires further step to generate a detectable signal. In some embodiments, the label is a soluble label, such as a colorimetric, radioactive, enzymatic, luminescent or fluorescent label. Exemplary fluorescent label includes Tide Fluor 5, and the DyLight Fluor family of fluorescent dyes, *e*.*g*., DyLight 350, DyLight 405, DyLight 488, DyLight 550, DyLight 594, DyLight 633, DyLight 650, DyLight 680, DyLight 755 and DyLight 800 produced by Dyomics in collaboration with Thermo Fisher Scientific. In other embodiments, the label is a particle or particulate label, such as a particulate direct label, or a colored particle label. Exemplary particle or particulate labels include colloidal gold label, latex particle label, electrochemical particle label, magnetic particle label, nanoparticle label and quantum dot label. Depending on the specific configurations, the labels such as colorimetric, radioactive, enzymatic, luminescent or fluorescent label, can be either a soluble label or a particle or particulate label.

The labeled reagent can be dried in the presence of a material that: a) stabilizes the labeled reagent; b) facilitates solubilization or resuspension of the labeled reagent in a liquid; and/or c) facilitates mobility of the labeled reagent. The exemplary material can be a protein, *e.g.*, a casein or BSA, a peptide, a polysaccharide, a sugar, a polymer, *e.g.*, polyvinylpyrrolidone (PVP-40), a gelatin, a detergent, *e.g.*, Tween-20, and a polyol, *e.g.*, mannitol. *See e.g.*, U.S. patent Nos. 5,120,643 and 6,187,598. In some embodiments, the labeled reagent, *e.g.*, a fluorescently labeled antibody, can be conjugated to polyethylene glycol (PEG) and/or polyethylene oxide (PEO). The presence of PEG and/or PEO can increase solubility, prolong stability and minimizes nonspecific binding of the labeled reagent. Although not to be bound by a particular theory, the presence of PEG and/or PEO can minimize nonspecific binding of the labeled reagent by causing the binding reagents or antibodies to sterically repel one another as well as other proteins and/or surfaces, *e*.*g*., surfaces of a container or the test device. PEG and/or PEO can be conjugated to the labeled reagent by any suitable ways. For example, PEG and/or PEO can be conjugated to the labeled reagent via various amines, *e*.*g*., primary amines, and/or sulfhydryl groups.

The test device can further comprise a control location for any suitable purpose. In some embodiments, a control location can comprise means for indicating proper flow of the liquid sample, means for indicating that the labeled reagent is added to the device and/or means for indicating that the labeled reagent is properly solubilized or dispersed, *e.g.*, a labeled reagent added by an operator and/or a labeled reagent embedded on a test device. The means can comprise a substance that will generate a detectable signal, *e*.*g*., fluorescent, color or electrical signal, once a liquid flow along or through the control location. For example, a labeled binding partner, *e.g.*, a labeled avidin or strepavidin, can be dried on the device. The labeled binding partner can be transported to a control location with an immobilized corresponding binding partner, *e*.*g*., biotin, to generate a detectable signal at the control location. The detection of the signal at the control location can be used to indicate proper addition and flow of sample or other liquid, and/or proper solubilization, suspension and transportation of the labeled reagents to the intended locations.

In other embodiments, a control location can comprise means for indicating a valid test result. In one example, the means comprises a binding reagent that binds to a binding reagent with a detectable label that also binds to the analyte. In another example, the means comprises a binding reagent that binds to a binding reagent with a detectable label that does not bind to the analyte. In still another example, the means comprises a binding reagent that binds to a substance in a test sample that is not a target analyte.

In still other embodiments, a control location can comprise means for indicating non-specific or unintended specific binding, or indicating heterophilic antibody interference, *e*.*g*., human anti-mouse antibody (HAMA) interference. In still other embodiments, a control location can comprise means for generating a control signal that is compared to signals at the test locations in determining amounts of the multiple analytes. The test device can comprise a single or multiple control locations, *e.g.,* a positive control location and a negative control location.

The analytes and/or the labeled reagent can be transported to the test locations by any suitable methods. In some embodiments, a sample liquid alone is used to transport the analytes and/or the labeled reagent to the test locations. In other embodiments, a developing liquid is used to transport the analytes and/or the labeled reagent to the test locations. In still other embodiments, a combination of a sample liquid and a developing liquid is used to transport the analytes and/or the labeled reagent to the test locations.

The test device can further comprise a housing that covers at least a portion of the test device, wherein the housing comprises a sample application port to allow sample application upstream from or to the test locations and an optic opening around the test locations to allow signal detection at the test locations. The optic opening can be achieved in any suitable way. For example, the optic opening can simply be an open space. Alternatively, the optic opening can be a transparent cover.

In some embodiments, the housing covers the entire test device. In other embodiments, at least a portion of the sample receiving portion of the matrix or the sample application element is not covered by the housing and a sample or a buffer diluent is applied to the portion of the sample receiving portion of the matrix or the sample application element outside the housing and is then transported to the test locations. The housing can comprise any suitable material. For example, the housing can comprise a plastic material. In another example, the housing, whether in part or in its entirety, can comprise an opaque, translucent and/or transparent material.

In one embodiment, the test device is configured for quantitatively detecting estradiol in a saliva sample from a subject ranging from about 1 pg/ml to about 30 pg/ml.

In any of the preceding embodiments, the test device can be configured for quantitatively detecting progesterone in a saliva sample from a subject ranging from about 50 pg/ml to about 500 pg/ml.

The present test device can be used for quantitatively detecting analytes with any desired or intended precision. In some embodiments, the present test device can be used for quantitatively detecting analytes, wherein the amount of at least one analyte, some analytes, or each of the analytes is determined with a CV ranging from about 0.1% to about 10%. Preferably, at least one analyte, some analytes, or each of the analytes has a concentration ranging from about 1 pg/ml to about 1 µg/ml, *e.g.,* about 1 pg/ml, 10 pg/ml, 100 pg/ml, 1 ng/ml, 2 ng/ml, 3 ng/ml, 3.5 ng/ml, 4 ng/ml, 5 ng/ml, 6 ng/ml, 7 ng/ml, 8 ng/ml, 9 ng/ml, 10 ng/ml, 100 ng/ml, 200 ng/ml, 300 ng/ml, 400 ng/ml, 500 ng/ml, 600 ng/ml, 700 ng/ml, 800 ng/ml, 900 ng/ml, 950 ng/ml, or higher.

The present test device can further comprise a liquid container. The liquid container can comprise any suitable liquid and/or reagent. For example, the liquid container can comprise a developing liquid, a wash liquid and/or a labeled reagent

The present test device can further comprise machine-readable information, *e*.*g*., a barcode. The barcode can comprise any suitable information. In some embodiments, the barcode comprises lot specific information of the test device, *e.g.,* lot number of the test device. In other embodiments, the machine-readable information is comprised in a storage medium, *e*.*g*., a (radio-frequency identification) RFID device. The RFID device can comprise any suitable information. For example, the RFID device comprises lot specific information, information on a liquid control or information to be used for quality control purpose.

In some embodiments, a fluorescent conjugate comprising a biological reagent and a fluorescent molecule is used to generate a detectable signal at the test locations. In this case, the fluorescent conjugate and/or the test device can further comprise a means for impeding phototoxic degradation of the biological reagent or impeding nonspecific binding of the fluorescent conjugate to the test device or a non-analyte moiety. Any suitable means or substances can be used to impede phototoxic degradation of the biological reagent. *See. e.g.*, U.S. Patent Nos. 6,544,797 and 7,588,908. For example, the means for impeding phototoxic degradation of the biological reagent can comprise a cross-linking substance having a long molecular distance, whereby the cross-linking substance links the fluorescent molecule and the biological reagent. In other examples, a protein; a quencher of singlet oxygen; a quencher of a free radical; a system for depleting oxygen; or a combination thereof can be used to impede phototoxic degradation of the biological reagent.

Any suitable means or substances can be used to impede nonspecific binding of the fluorescent conjugate. For example, the means for impeding nonspecific binding of the fluorescent conjugate comprises PEG or PEO bound to the fluorescent conjugate.

The test device tis used in a competition assay format, generally described in the following. An analyte or analyte analog is used as a capture reagent at the test location. A labeled reagent, *e.g.*, an antibody having a detectable label, is either added in a liquid or previously dried on the test device and redissolved or resuspnded by a liquid. An analyte in a sample will compete with the analyte or analyte analog at the test location for binding to the labeled reagent, *e.g.*, an antibody, having a detectable label. Typically, different analytes or analyte analogs are used at different test locations to compete with different analytes for binding to the different labeled reagents.

Antibodies used in the immunoassays described herein preferably specifically bind to the target analytes, estradiol and progesterone. The term "specifically binds" is not intended to indicate that an antibody binds exclusively to its intended target since, as noted above, an antibody binds to any polypeptide displaying the epitope(s) to which the antibody binds. In some cases, an antibody "specifically binds" if its affinity for its intended target is about 5-fold greater when compared to its affinity for a non-target molecule which does not display the appropriate epitope(s). Preferably the affinity of the antibody may be at least about 5 fold, preferably 10 fold, more preferably 25-fold, even more preferably 50-fold, and most preferably 100-fold or more, greater for a target molecule than its affinity for a non-target molecule. In preferred embodiments, preferred antibodies bind with affinities of at least about 10⁷ M⁻¹, and preferably between about 10⁸ M⁻¹ to about 10⁹ M⁻¹, about 10⁹ M⁻¹ to about 10¹⁰ M⁻¹, or about 10¹⁰ M⁻¹ to about 10¹² M⁻¹ .

Affinity is calculated as K_{d} = k_{off}/kₒₙ (k_{off} is the dissociation rate constant, Kₒₙ is the association rate constant and K_{d} is the equilibrium constant). Affinity can be determined at equilibrium by measuring the fraction bound (r) of labeled ligand at various concentrations (c). The data are graphed using the Scatchard equation: r/c = K(n-r): where r = moles of bound ligand/mole of receptor at equilibrium; c = free ligand concentration at equilibrium; K = equilibrium association constant; and n = number of ligand binding sites per receptor molecule. By graphical analysis, r/c is plotted on the Y-axis versus r on the X-axis, thus producing a Scatchard plot. Antibody affinity measurement by Scatchard analysis is well known in the art. *See, e.g.*, van Erp et al., J. Immunoassay 12: 425-43, 1991; Nelson and Griswold, Comput. Methods Programs Biomed. 27: 65-8, 1988.

Numerous publications discuss the use of phage display technology to produce and screen libraries of polypeptides for binding to a selected analyte. *See, e.g*, Cwirla et al., Proc. Natl. Acad. Sci. USA 87, 6378-82, 1990; Devlin et al., Science 249, 404-6, 1990, Scott and Smith, Science 249, 386-88, 1990; and Ladner et al., U.S. Pat. No. 5,571,698. A basic concept of phage display methods is the establishment of a physical association between DNA encoding a polypeptide to be screened and the polypeptide. This physical association is provided by the phage particle, which displays a polypeptide as part of a capsid enclosing the phage genome which encodes the polypeptide. The establishment of a physical association between polypeptides and their genetic material allows simultaneous mass screening of very large numbers of phage bearing different polypeptides. Phage displaying a polypeptide with affinity to a target bind to the target and these phage are enriched by affinity screening to the target. The identity of polypeptides displayed from these phage can be determined from their respective genomes. Using these methods a polypeptide identified as having a binding affinity for a desired target can then be synthesized in bulk by conventional means. *See, e.g.*, U.S. Patent No. 6,057,098.

The antibodies that are generated by these methods may then be selected by first screening for affinity and specificity with the purified polypeptide of interest and, if required, comparing the results to the affinity and specificity of the antibodies with polypeptides that are desired to be excluded from binding. The screening procedure can involve immobilization of the purified polypeptides in separate wells of microtiter plates. The solution containing a potential antibody or groups of antibodies is then placed into the respective microtiter wells and incubated for about 30 min to 2 h. The microtiter wells are then washed and a labeled secondary antibody (for example, an anti-mouse antibody conjugated to alkaline phosphatase if the raised antibodies are mouse antibodies) is added to the wells and incubated for about 30 min and then washed. Substrate is added to the wells and a color reaction will appear where one or more antibodies to the immobilized polypeptide(s) are present.

The antibodies so identified may then be further analyzed for affinity and specificity in the assay design selected. In the development of immunoassays for a target protein, the purified target protein acts as a standard with which to judge the sensitivity and specificity of the immunoassay using the antibodies that have been selected. Because the binding affinity of various antibodies may differ; certain antibody pairs *(e.g.,* in sandwich assays) may interfere with one another sterically, etc., assay performance of an antibody may be a more important measure than absolute affinity and specificity of an antibody.

While the present application describes antibody-based binding assays in detail, alternatives to antibodies as binding species in assays are well known in the art. These include receptors for a particular target, aptamers, etc. Aptamers are oligonucleic acid or peptide molecules that bind to a specific target molecule. Aptamers are usually created by selecting them from a large random sequence pool, but natural aptamers also exist. High-affinity aptamers containing modified nucleotides conferring improved characteristics on the ligand, such as improved in vivo stability or improved delivery characteristics. Examples of such modifications include chemical substitutions at the ribose and/or phosphate and/or base positions, and may include amino acid side chain functionalities.

In some embodiments, the present invention provides for a test device wherein a liquid has moved laterally along the test device to generate a detectable signal at the test locations.

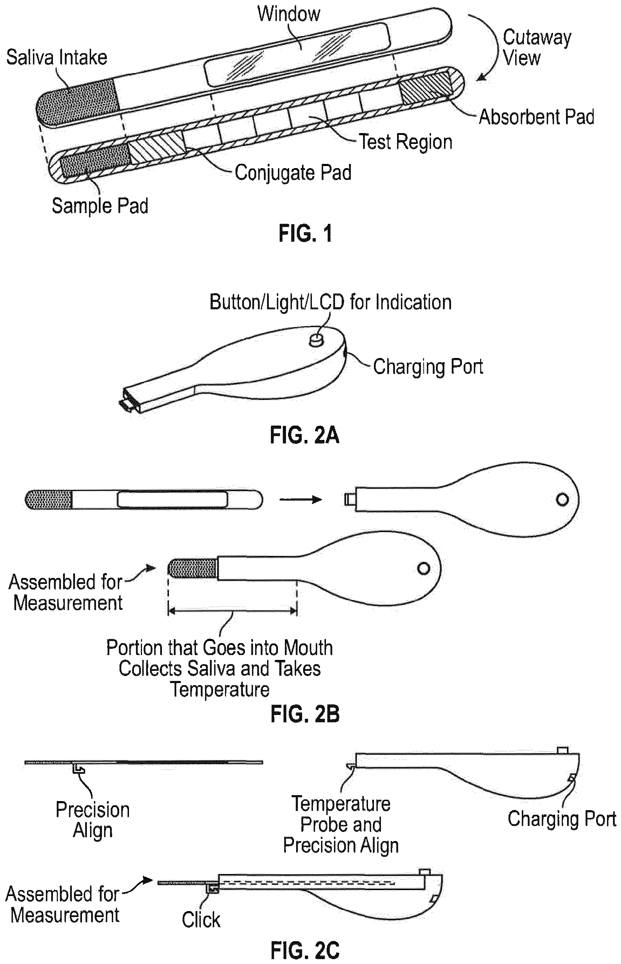

**Figure 1** illustrates a lateral flow assay test strip which is embedded within a cartridge. The cartridge may take one of many forms (plastic, laminated, other novel material, *etc*.). In this non-limiting example, this entire piece of our product is designed to be single use and disposable. In this example, the cartridge has a portion at one end where saliva can enter the cartridge through a matrix of pores and absorbs into a sample pad. This end of the cartridge is intended in this example to be inserted into the mouth under the tongue. The saliva then wicks through sample pad, conjugate pad, and test region, and is collected by the absorbent pad at the opposite end. The window in the cartridge allows light to pass freely in and out, providing for the test to be read by the electronic reader.

**Figure 2A** illustrates the electronic reader according to one embodiment of the present disclosure. **Figure 2B** illustrates that the lateral flow assay cartridge is designed to slide into the reader so that the window in the cartridge aligns with electronics that will read the results from the test strip. The "click" mechanism provides for this alignment when the user slides the cartridge into the reader. The cartridge is "unclicked" when testing is done and the cartridge can be thrown away. In contrast, the reader is a reusable product. **Figure 2C** illustrates that in this example, the neck portion of the reader is designed to insert into the mouth under the tongue, with the saliva-collection portion of the cartridge displayed. The portion that inserts into the mouth, possibly associated with the small "click" protrusion on the reader, has a thin thermocouple wire embedded that will allow the device to take a user's basal temperature while the lateral flow assay is collecting saliva.

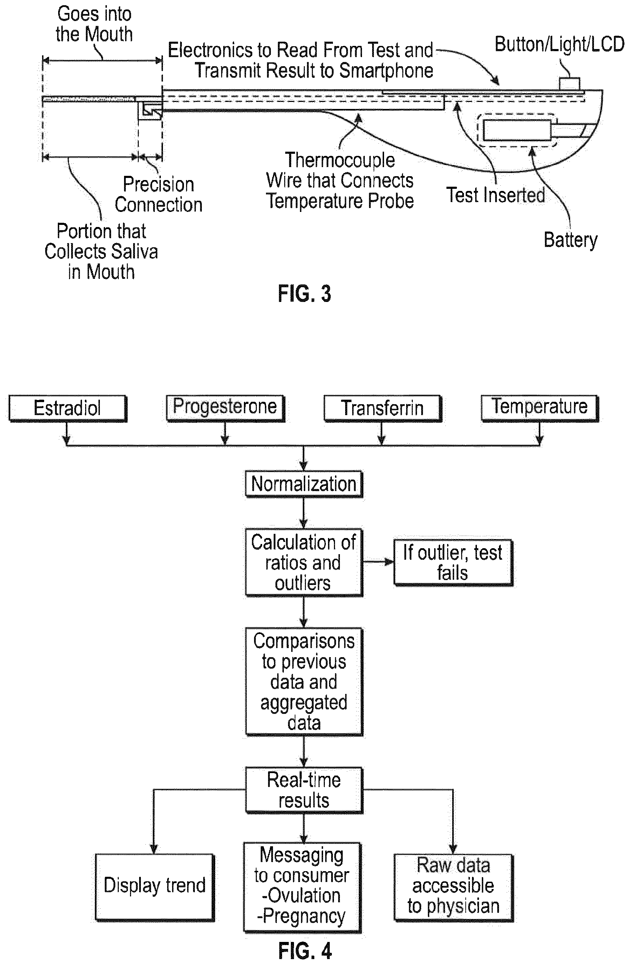

**Figure 3** illustrates that the electronic reader reads results from the lateral flow assay. In this embodiment, the cartridge inserts into the body of the reader so that the cartridge window aligns with the appropriate electronics to sense results from the test strip (it clicks into place to assure accurate placement). The neck portion of the reader fits into the mouth with the saliva collection portion under the tongue. The reader can also take a user's basal temperature while inserted into the mouth. This is achieved by a thermocouple wire embedded in the neck portion of the reader, possibly within the precision "click" protrusion. The reader has a battery which is rechargeable through a port in the body of the reader.

The operation of the device disclosed herein can comprise: (1) removing a disposable test cartridge from packaging; (2) inserting the test cartridge into a reader until it clicks into place; (3) the reader automatically turning on after the click; (4) the reader automatically attempting to interface with an mobile app and setup connection; (5) an indicator light on the reader turning on, indicating that the test is ready and a new test cartridge is in place; (6) the mobile app prompting an user to place the neck portion of the assembled reader/test into the mouth under tongue; (7) the user placing the neck portion of the assembly into his or her mouth under the tongue; (8) the app controlling the reader to sense saliva volume adequacy, for example, based upon a normalization scheme disclosed herein; (9) meanwhile, the app also retrieving basal temperature information from the reader; (10) when saliva analysis and temperature measurement are complete, the app prompting the user to remove the test strip from the mouth, (11) the reader beeping and/or indicator light telling the user to remove the test strip from the mouth, (12) the user prompted to set assembly aside without removing the test cartridge, and in proximity to the device with the app; (12) app controlling continued test reading and indicator light indicating test is in process; (13) when complete, app displaying appropriate data/messaging and prompts the user to discard the test cartridge; (14) the indicator light turning off, and then the reader automatically turning off; and (15) the user interacting with their results using the app.

The device can comprise a LCD located by the indicator light. In other embodiments, the device comprises a LCD instead of the indicator light, and the information given to the user by the indicator light is instead displayed on the LCD. In one aspect, the LCD can indicate to the user whether the cartridge is inserted correctly, whether the wireless connection is established, and/or whether the assay is completed.

Disclosed herein is also a kit for quantitatively detecting multiple analytes in a sample, which kit comprises a test device as described above. The kit can further comprise an instruction for using the test device to quantitatively detect the multiple analytes in a sample, and/or means for obtaining and/or processing the sample to be tested.

### C. Methods for quantitatively detecting multiple analytes in a sample

In another aspect, the present invention provides a method for quantitatively detecting 17-beta estradiol and progesterone in a saliva sample, *e.g.,* a saliva sample from a subject, which method comprises: a) contacting a saliva sample with the test device disclosed herein, wherein the saliva sample is applied to a site of the test device upstream of the first and second test location(s), wherein the site optionally comprises an absorbent material, *e*.*g*., a polymeric material and/or a cellulosic material; b) transporting 17-beta estradiol and progesterone, if present in the saliva sample, and the labeled Fab fragment that specifically binds to 17-beta estradiol and the labeled Fab fragment that specifically binds to progesterone to the two first test locations on the first porous matrix, and transporting the Fab fragment that specifically binds to the normalization substance to the second test location; and c) assessing the two first detectable signals at the two first test locations, wherein: 1) the second detectable signal(s) is assessed and compared to the first detectable signal(s) to assess levels of 17-beta estradiol and progesterone in said saliva sample. In an embodiment, said method further comprises measuring temperature of a subject while the device is inserted into the mouth of the subject. In one aspect, the site of the test device upstream of the test location(s) is a saliva collector site of the device. In one embodiment, the saliva collector site is engineered to promote unidirectional flow of saliva toward another site of the device, for example, a test location on the device.

The liquid sample and the labeled reagent can be premixed to form a mixture and the mixture is applied to the test device. The labeled reagent can be stored and/or used in any suitable manner. For example, the labeled reagent can be stored and/or used in liquid format. Alternatively, the labeled reagent can be stored in a dry format off the device, *e.g.,* in a container, pipette tip, or tube. For example, the labeled reagent can be dried on the surface of the container, pipette tip, or tube. In another example, the labeled reagent can be dried as particles or beads and the particles or beads can be stored in the container, pipette tip, or tube. In use, the dried labeled reagent, either dried on the surface of the container, pipette tip, or tube, or dried as particles or beads, can be dissolved or resuspended by a liquid sample or buffer to form a mixture and the mixture is applied to the test device. In other embodiments, the present method can further comprise a washing step after the mixture is applied to the test device. The washing step can be conducted by any suitable ways. For example, the washing step can comprise adding a washing liquid after the mixture is applied to the test device. In another example, the test device can comprise a liquid container comprising a washing liquid and the washing step comprises releasing the washing liquid from the liquid container. *See e.g.*, U.S. patent No. 4,857,453.

The test device can also comprise a dried labeled reagent before use and the dried labeled reagent can be solubilized or resuspended, and transported to the test locations by the liquid sample. In some embodiments, the dried labeled reagent is located downstream from the sample application site, and the dried labeled reagent is solubilized or resuspended, and transported to the test location by the liquid sample. In other embodiments, the dried labeled reagent is located upstream from the sample application site, and the dried labeled reagent is solubilized or resuspended, and transported to the test location by another liquid. In still other embodiments, multiple analytes and/or labeled reagent(s) are solubilized or resuspended, and transported to the test location by the liquid sample alone. In yet other embodiments, multiple analytes and/or labeled reagent(s) are solubilized or resuspended, and transported to the test location by another liquid, or by a combination of the sample liquid and another liquid, *e.g.,* a developing fluid.

The present method can be used for quantitatively detecting the multiple analytes in any suitable sample. In some embodiments, the sample is a biological sample or clinical sample.

Depending on the assay format and the label used in the method, the detectable signal can be assessed by any suitable methods. For example, when the label is a visual direct label, *e.g.,* a gold or latex particle label, the detectable signal can be assessed by naked eyes without using any instrument. In other examples, the detectable signal is often or typically assessed by a reader, such as an optical reader, an electronic reader, a magnetic reader, or an electrochemical reader, or a combination thereof. In many cases, a reader is used to assess the detectable signal regardless whether the detectable signal can be assessed by naked eyes or not. For example even if a visual direct label is used, the detectable signal is often or typically assessed by a reader for quantitatively detecting the analytes.

In some embodiments, the detectable signal is a fluorescent signal and the fluorescent signal is assessed by a fluorescent reader. Depending on the assay format and the fluorescent label used in the method, any suitable fluorescent reader can be used. For example, the fluorescent reader can be a laser based or a light emitting diode (LED) based fluorescent reader.

The fluorescent reader can illuminate at any suitable angle relative to the surface of the test device to excite the fluorescent label at the test locations and/or can detect the fluorescent light at any suitable angle relative to the surface of the test device. In some embodiments, the fluorescent reader illuminates at an angle substantially normal, or normal, to the surface of the test device to excite the fluorescent label at the test locations and/or detects the fluorescent light at an angle substantially normal, or normal, to the surface of the test device. In other embodiments, the surface for detection of the fluorescent light in the fluorescent reader is substantially parallel, or parallel, to the surface of the test device. In still other embodiments, the surface for detection of the fluorescent light in the fluorescent reader is not parallel to the surface of the test device. A light source and a photodetector can be positioned at the same side or different sides of the test device.

An illumination system of the reader can scan any suitable or desired size or defined area of the test and/or control locations to detect the detectable or fluorescent signal. In some embodiments, at least one, some or each of the test locations comprises a capture region characterized by a first dimension transverse to the lateral flow direction and a second dimension parallel to the lateral flow direction, and the reader comprises an illumination system operable to focus a beam of light onto an area of the test and/or control locations having at least one surface dimension at most equal to smallest of the first and second dimensions of the test and/or control locations.

The reader can comprise a single or multiple photodetectors. The detectable signal can be measured at any suitable or desired time point(s). In some embodiments, the detectable signal is measured before the detectable signal reaches its equilibrium. In other embodiments, the detectable signal is measured after the detectable signal reaches its equilibrium. In still other embodiments, the detectable signal is measured at a preset time after the sample is added to the test device.

The present methods can further comprise comparing the amounts of the multiple analytes to a single threshold, multiple thresholds or a reference range, *e.g.,* a normal range, a disease range, a clinical range, or a reference range based on calibrated or uncalibrated analyte levels or concentrations. In some embodiments, the amount of at least one, some or each of the multiple analytes is compared to a single corresponding threshold or multiple corresponding thresholds. In other embodiments, the amounts of the multiple analytes are used to form a composite amount that is compared to a composite threshold or reference range.

The present methods can be used for quantitatively detecting any suitable number of analytes. For example, the present methods can be used for quantitatively detecting 2, 3, 4, 5, 6, 7, 8, 9, 10 or more analytes. The present methods can be used for any suitable purpose. For example, the present can be used for quantitatively detecting multiple analytes that are diagnostic, prognostic, risk assessment, stratification and/or treatment monitoring markers.

In other embodiments, the present methods can be used for quantitatively detecting any suitable markers for assessing hormone(s), ovulation, pregnancy, fertility, *e*.*g*., hormonal, ovulation, pregnancy, fertility status, time window, trend, or therapy monitoring or guidance.

### D. Kits and systems for quantitatively detecting an analyte in a sample

Disclosed herein is a kit for quantitatively detecting 17-beta estradiol and progesterone in a saliva sample, *e.g.,* a saliva sample from a subject, which kit comprises: a) a test device of any of the preceding embodiments; and b) an instruction for using the test device for quantitatively detecting an analyte in a saliva sample.

In one aspect, the present invention provides a system for quantitatively detecting the multiple analytes 17-beta estradiol and progesterone in a sample, which system comprises: a) a test device described above; and b) a reader that comprises a light source and a photodetector to detect a detectable signal.

Depending on the assay format and the label used in the assay, any suitable reader can be used, *e.g.,* a fluorescent reader. Depending on the assay format and the fluorescent label used in the method, any suitable fluorescent reader can be used. For example, the fluorescent reader can be a laser based or a light emitting diode (LED) based fluorescent reader.

The fluorescent reader can illuminate at any suitable angle relative to the surface of the test device to excite the fluorescent label at the test locations and/or can detect the fluorescent light at any suitable angle relative to the surface of the test device. In some embodiments, the fluorescent reader illuminates at an angle substantially normal, or normal, to the surface of the test device to excite the fluorescent label at the test locations and/or detects the fluorescent light at an angle substantially normal, or normal, to the surface of the test device. In other embodiments, the surface for detection of the fluorescent light in the fluorescent reader is substantially parallel, or parallel, to the surface of the test device. In still other embodiments, the surface for detection of the fluorescent light in the fluorescent reader is not parallel to the surface of the test device. A light source and a photodetector can be positioned at the same side or different sides of the test device.

An illumination system of the reader can scan any suitable or desired size or defined area of the test and/or control locations to detect the detectable or fluorescent signal. In some embodiments, at least one, some or each of the test locations comprises a capture region characterized by a first dimension transverse to the lateral flow direction and a second dimension parallel to the lateral flow direction, and the reader comprises an illumination system operable to focus a beam of light onto an area of the test and/or control locations having at least one surface dimension at most equal to smallest of the first and second dimensions of the test and/or control locations.

The reader can comprise a single or multiple photodetectors. The detectable signal can be measured at any suitable or desired time point(s). In some embodiments, the detectable signal is measured before the detectable signal reaches its equilibrium. In other embodiments, the detectable signal is measured after the detectable signal reaches its equilibrium. In still other embodiments, the detectable signal is measured at a preset time after the sample is added to the test device.

The present systems can comprise machine-readable information and a reader for detecting the machine-readable information. For example, the test device can comprise machine-readable information, *e*.*g*., a barcode, and the reader can comprise a function for detecting the machine-readable information, *e*.*g*., a barcode reader. The machine-readable information can be any suitable or desired information, *e*.*g*., lot specific information of the test device or the assay, information on a liquid control or information to be used for quality control purpose, *etc.* In some embodiments, the present system, *e.g.*, the present device, can comprise a barcode that comprises lot specific information of the test device, *e.g.*, lot number of the test device. In other embodiments, the present system can comprise a storage medium, *e*.*g*., a RFID device. The RFID device can comprise lot specific information, information on a liquid control or information to be used for quality control purpose. The RFID device can be provided in any suitable ways or locations. For example, an RFID device can be provided as an RFID card with an embedded antenna and an RFID tag. In another example, the RFID device or card can be provided within a package of a plurality of the present devices, or can be provided on the package, but is not made part of a present device. In still another example, the RFID device or card can be provided on any suitable location on a test device, *e.g.,* on the housing of the test device or at any location that is not test locations.

The present systems can be used for quantitatively detecting 17-beta estradiol and progesterone. For example, the kit and/or the system can be used for assessing hormone(s), ovulation, pregnancy, fertility, *e*.*g*., hormonal, ovulation, pregnancy, fertility status, time window, trend, or therapy monitoring or guidance. In one embodiment, the method is used for predicting ovulation. In one embodiment, the method is used for confirming pregnancy. In one embodiment, the kit and/or the system can be used for assessing overall fertility and/or ability to conceive, for family planning, and/or for birth control.

## Claims

1. A lateral flow test device for quantitatively detecting 17-beta estradiol and progesterone in a saliva sample, *e.g.,* a saliva sample from a subject, which device comprises:
a first porous matrix that comprises two first test locations on said first porous matrix, one of said first test locations comprising a test reagent being able to compete with 17-beta estradiol in said sample for binding to a labeled Fab fragment that specifically binds to 17-beta estradiol, and the other said first test location comprising a test reagent being able to compete with progesterone in said sample for binding to a labeled Fab fragment that specifically binds to progesterone, wherein the saliva sample can flow laterally along said test device and can pass said two first test locations to form two first detectable signals, wherein:
a) said device further comprises a second porous matrix that comprises a second test location on said second porous matrix, said second test location comprising a second test reagent that is a normalization substance that is able to compete with a normalization substance in said saliva sample for binding to a binding reagent for said normalization substance, wherein the normalization substance is transferrin, albumin, creatinine, or a combination thereof, wherein a labeled Fab fragment that specifically binds to the normalization substance is dried on the test device, and wherein the saliva sample can flow laterally along said test device and can pass said second test location to form a second detectable signal, and said first two detectable signals and said second detectable signal are able to be compared to assess levels of 17-beta estradiol and progesterone in said saliva sample; and
b) said levels of 17-beta estradiol and progesterone in the saliva sample can be determined and can be combined into a single test result;
c) said first porous matrix and said second porous matrix are the same porous matrix; and
d) wherein the labeled Fab fragment that specifically binds to 17-beta estradiol, the labeled Fab fragment that specifically binds to progesterone, and the labeled fragment that specifically binds to the normalization substance, are each dried on the test device upstream from the first and second test locations.

2. The test device of claims 1, which is configured for quantitatively detecting 17-beta estradiol in the saliva sample from a subject ranging from 1 pg/mL to 30 pg/mL.

3. The test device of any one of claims 1 or 2, which is configured for quantitatively detecting progesterone in the saliva sample from a subject ranging from 50 pg/mL to 500 pg/mL.

4. The test device of any one of claims 1 to 3, which is configured for assessing ovulation, pregnancy, fertility, fertility status, time window, trend, therapy monitoring or guidance, wherein the test device is optionally configured for predicting ovulation, for confirming pregnancy, or for assessing overall fertility and/or ability to conceive.

5. The test device of any one of claims 1-4, wherein the device is configured for the labeled Fab fragments to be solubilized or resuspended by the saliva sample.

6. The test device of claim 5, wherein the device is configured for the solubilized or resuspended labeled Fab fragments to be transported to the test locations by the saliva sample.

7. The test device of any of claims 1-6, which further comprises machine-readable information, *e*.*g*., a barcode.

8. A method for quantitatively detecting 17-beta estradiol and progesterone in a saliva sample, *e.g.*, a saliva sample from a subject, which method comprises:
a) contacting a saliva sample with the test device of any of claims 1 to 7, wherein the saliva sample is applied to a site of the test device upstream of the first and second test locations, wherein the site optionally comprises an absorbent material, *e*.*g*., a polymeric material and/or a cellulosic material;
b) transporting 17-beta estradiol and progesterone, if present in the saliva sample, and the labeled Fab fragment that specifically binds to 17-beta estradiol and the labeled Fab fragment that specifically binds to progesterone to the two first test locations on the first porous matrix, and transporting the Fab fragment that specifically binds to the normalization substance to the second test location; and
c) assessing the first two detectable signals at the two first test locations on the first porous matrix,
wherein:
i) the second detectable signal at the second test location on the second porous matrix is assessed and compared to the two first detectable signals to assess levels of 17-beta estradiol and progesterone in said saliva sample; and
ii) combining the levels of 17-beta estradiol and progesterone in the saliva sample into a single test result.

9. The method of claim 8, wherein the dried, labeled Fab fragments are solubilized or resuspended, and transported to the two test locations on the first porous matrix by the saliva sample

10. The method of any of claims 8 or 9, which is used for assessing ovulation, pregnancy, fertility, fertility status, time window, trend, therapy monitoring or guidance.

11. The method of claim 10, which is used for predicting ovulation, used for confirming pregnancy, or used for assessing overall fertility and/or ability to conceive, for family planning, and/or for birth control.

## Patentansprüche

1. Test-Vorrichtung für Lateral-Flow Analysen zur quantitativen Detektion von 17-beta-Östradiol und Progesteron in einer Speichelprobe, z.B. einer Speichelprobe eines Subjekts, wobei die Vorrichtung aufweist:
eine erste poröse Matrix, welche zwei erste Test-Abschnitte auf der porösen Matrix aufweist, wobei einer der Test-Abschnitte ein Test-Reagenz aufweist, das dazu in der Lage ist, mit 17-beta-Östradiol in der Probe um die Bindung an ein spezifisch 17-beta-Östradiol bindendes gelabeltes Fab-Fragment zu konkurrieren, und der andere erste Test-Abschnitt ein Test-Reagenz aufweist, das dazu in der Lage ist, mit Progesteron in der Probe um die Bindung an ein spezifisch Progesteron bindendes gelabeltes Fab-Fragment zu konkurrieren, wobei die Speichelprobe lateral entlang der Test-Vorrichtung fließen kann und die zwei ersten Test-Abschnitte passieren kann, sodass zwei erste detektierbare Signale entstehen, wobei:
a) die Vorrichtung ferner eine zweite poröse Matrix aufweist, welche einen zweiten Test-Abschnitt auf der zweiten porösen Matrix aufweist, wobei der zweite Test-Abschnitt ein zweites Test-Reagenz aufweist, welches eine Normalisierungssubstanz ist, die dazu in der Lage ist, mit einer Normalisierungssubstanz in der Speichelprobe um die Bindung an ein Bindungsreagenz für die Normalisierungssubstanz zu konkurrieren, wobei die Normalisierungssubstanz Transferrin, Albumin, Creatinin oder eine Kombination aus diesen ist, wobei ein spezifisch an die Normalisierungssubstanz bindendes gelabeltes Fab-Fragment auf die Test-Vorrichtung angetrocknet ist, und wobei die Speichelprobe lateral entlang der Test-Vorrichtung fließen kann und den zweiten Test-Abschnitt passieren kann, sodass ein zweites detektierbares Signal entsteht, und wobei die zwei ersten detektierbaren Signale und das zweite detektierbare Signal verglichen werden können, um die Level an 17-beta-Östradiol und Progesteron in der Speichelprobe zu quantifizieren; und
b) die Level an 17-beta-Östradiol und Progesteron in der Speichelprobe bestimmt und in einem einzigen Testergebnis kombiniert werden können;
c) die erste poröse Matrix und die zweite poröse Matrix dieselbe poröse Matrix sind; und
d) wobei das spezifisch 17-beta-Östradiol bindende gelabelte Fab-Fragment, das spezifisch Progesteron bindende gelabelte Fab-Fragment und das spezifisch die Normalisierungssubstanz bindende gelabelte Fab-Fragment auf der Test-Vorrichtung angetrocknet sind stromaufwärts von den ersten und dem zweiten Test-Abschnitten.

2. Test-Vorrichtung nach Anspruch 1, die zur quantitativen Detektion von 17-beta-Östradiol in der Speichelprobe eines Subjekts in einer Konzentration von 1 pg/mL bis 30 pg/mL konfiguriert ist.

3. Test-Vorrichtung nach einem der Ansprüche 1 oder 2, die zur quantitativen Detektion von Progesteron in der Speichelprobe eines Subjekts in einer Konzentration von 50 pg/mL bis 500 pg/mL konfiguriert ist.

4. Test-Vorrichtung nach einem der Ansprüche 1 bis 3, die zur Abschätzung der Ovulation, der Schwangerschaft, der Fertilität, des Fertilitätsstatus, -zeitfensters, -trends, zur Fertiliätstherapiebegleitung oder zur Fertilitätstherapieleitung konfiguriert ist, wobei die Test-Vorrichtung optional konfiguriert ist zur Vorhersage der Ovulation, zur Bestätigung von Schwangerschaft, oder zur Abschätzung der allgemeinen Fertilität und/oder Empfängnisfähigkeit.

5. Test-Vorrichtung nach einem der Ansprüche 1 bis 4, wobei die Test-Vorrichtung dazu konfiguriert ist, dass die gelabelten Fab-Fragmente durch die Speichelprobe solubilisiert oder resuspendiert werden.

6. Test-Vorrichtung nach Anspruch 5, wobei die Test-Vorrichtung dazu konfiguriert ist, dass die solubilisierten oder resuspendierten Fab-Fragmente durch die Speichelprobe zu den Test-Abschnitten transportiert werden.

7. Test-Vorrichtung nach einem der Ansprüche 1 bis 6, die ferner maschinenlesbare Informationen aufweist, wie beispielsweise einen Barcode.

8. Verfahren zur quantitativen Detektion von 17-beta-Östradiol und Progesteron in einer Speichelprobe, z.B. einer Speichelprobe eines Subjekts, wobei das Verfahren aufweist:
a) In-Kontakt-Bringen einer Speichelprobe mit der Test-Vorrichtung nach einem der Ansprüche 1 bis 7, wobei die Speichelprobe in einem Abschnitt stromaufwärts von den ersten und dem zweiten Test-Abschnitten auf die Test-Vorrichtung aufgebracht wird, wobei der Abschnitt optional ein absorbierendes Material, wie z.B. ein polymerartiges und/oder zelluloseartiges Material aufweist;
b) Transportieren von 17-beta-Östradiol und Progesteron, sofern vorhanden in der Speichelprobe, des spezifisch 17-beta-Östradiol bindenden gelabelten Fab-Fragments und des spezifisch Progesteron bindenden gelabelten Fab-Fragments zu den zwei ersten Test-Abschnitten auf der ersten porösen Matrix und das Transportieren des spezifisch die Normalisierungssubstanz bindenden gelabelten Fab-Fragments zum zweiten Test-Abschnitt; und
c) Bestimmen der ersten zwei detektierbaren Signale an den zwei ersten Test-Abschnitten auf der ersten porösen Matrix, wobei:
i.) das zweite detektierbare Signal am zweiten Test-Abschnitt auf der zweiten porösen Matrix bestimmt wird und mit den zwei ersten detektierbaren Signalen verglichen wird, sodass die Level an 17-beta-Östradiol und Progesteron in der Speichelprobe bestimmt werden; und
ii.) Kombinieren der Level an 17-beta-Östradiol und Progesteron in der Speichelprobe in ein einziges Testergebnis.

9. Verfahren nach Anspruch 8, wobei die angetrockneten, gelabelten Fab-Fragmente durch die Speichelprobe solubilisiert oder resuspendiert und zu den zwei Test-Abschnitten auf der ersten porösen Matrix transportiert werden.

10. Verfahren nach einem der Ansprüche 8 oder 9, das zur Abschätzung der Ovulation, der Schwangerschaft, der Fertilität, des Fertilitätsstatus, -zeitfensters, -trends, Fertiliätstherapiebegleitung oder-Fertilitätstherapieleitung eingesetzt wird.

11. Verfahren nach Anspruch 10, das zur Vorhersage der Ovulation, Bestätigung von Schwangerschaft, Abschätzung der allgemeinen Fertilität und/oder Empfängnisfähigkeit, Familienplanung oder Geburtenkontrolle eingesetzt wird.

## Revendications

1. Dispositif de test à écoulement latéral pour la détection de manière quantitative de 17-bêta estradiol et de progestérone dans un échantillon de salive, *par ex.,* un échantillon de salive provenant d'un sujet, lequel dispositif comprenant :
une première matrice poreuse qui comprend deux premiers emplacements de test sur ladite première matrice poreuse, l'un desdits premiers emplacements de test comprenant un réactif de test qui est capable d'entrer en compétition avec le 17-bêta estradiol dans ledit échantillon pour la liaison à un fragment de Fab marqué qui se lie spécifiquement au 17-bêta estradiol, et l'autre dit premier emplacement de test comprenant un réactif de test qui est capable d'entrer en compétition avec la progestérone dans ledit échantillon pour la liaison à un fragment de Fab marqué qui se lie spécifiquement à la progestérone, l'échantillon de salive pouvant s'écouler de manière latérale le long dudit dispositif de test et pouvant passer lesdits deux premiers emplacements de test pour former deux premiers signaux détectables,
a) ledit dispositif comprenant en outre une deuxième matrice poreuse qui comprend un deuxième emplacement de test sur ladite deuxième matrice poreuse, ledit deuxième emplacement de test comprenant un deuxième réactif de test qui est une substance de normalisation qui est capable d'entrer en compétition avec une substance de normalisation dans ledit échantillon de salive pour la liaison à un réactif de liaison pour ladite substance de normalisation, la substance de normalisation étant la transferrine, l'albumine, la créatinine ou une combinaison correspondante, un fragment de Fab marqué qui se lie spécifiquement à la substance de normalisation étant séché sur le dispositif de test, et l'échantillon de salive pouvant s'écouler de manière latérale le long dudit dispositif de test et pouvant passer ledit deuxième emplacement de test pour former un deuxième signal détectable, et lesdits premiers deux signaux détectables et ledit deuxième signal détectable étant capables d'être comparés pour évaluer des taux de 17-bêta estradiol et de progestérone dans ledit échantillon de salive ; et
b) lesdits taux de 17-bêta estradiol et de progestérone dans l'échantillon de salive pouvant être déterminé et pouvant être combinés en un unique résultat de test ;
c) ladite première matrice poreuse et ladite deuxième matrice poreuse étant la même matrice poreuse ; et
d) le fragment de Fab marqué qui se lie spécifiquement au 17-bêta estradiol, le fragment de Fab marqué qui se lie spécifiquement à la progestérone, et le fragment marqué qui se lie spécifiquement à la substance de normalisation étant chacun séchés sur le dispositif de test en amont des premier et deuxième emplacements de test.

2. Dispositif de test selon la revendication 1, qui est configuré pour détecter quantitativement le 17-bêta estradiol dans l'échantillon de salive provenant d'un sujet dans une plage de 1 pg/mL à 30 pg/mL.

3. Dispositif de test selon la revendication 1 ou 2, qui est configuré pour détecter quantitativement la progestérone dans l'échantillon de salive provenant d'un sujet dans une plage de 50 pg/mL à 500 pg/mL.

4. Dispositif de test selon l'une quelconque des revendication 1 à 3, qui est configuré pour évaluer une ovulation, une grossesse, la fertilité, l'état de fertilité, la fenêtre temporelle, une tendance, un suivi ou un guidage de thérapie, le dispositif de test étant éventuellement configuré pour prédire une ovulation, pour confirmer une grossesse, ou pour évaluer la fertilité globale et/ou la capacité à concevoir.

5. Dispositif de test selon l'une quelconque des revendication 1 à 4, le dispositif étant configuré pour que les fragments de Fab marqués soient solubilisés ou remis en suspension par l'échantillon de salive.

6. Dispositif de test selon la revendication 5, le dispositif étant configuré pour que les fragments de Fab marqués solubilisés ou remis en suspension soient transportés vers les emplacements de test par l'échantillon de salive.

7. Dispositif de test selon l'une quelconque des revendication 1 à 6, qui comprend en outre des informations lisibles par machine, *par ex.*, un code-barre.

8. Procédé pour la détection de manière quantitative de 17-bêta estradiol et de progestérone dans un échantillon de salive, *par ex.*, un échantillon de salive provenant d'un sujet, lequel procédé comprenant :
a) la mise en contact d'un échantillon de salive avec le dispositif de test selon l'une quelconque des revendications 1 à 7, l'échantillon de salive étant appliqué sur un site du dispositif de test en amont des premier et deuxième emplacements de test, le site comprenant éventuellement un matériau absorbant, *par ex.*, un matériau polymérique et/ou un matériau cellulosique ;
b) le transport de 17-bêta estradiol et de progestérone, s'ils sont présents dans l'échantillon de salive, et du fragment de Fab marqué qui se lie spécifiquement au 17-bêta estradiol et du fragment de Fab marqué qui se lie spécifiquement à la progestérone vers les deux premiers emplacements de test sur la première matrice poreuse, et le transport du fragment de Fab qui se lie spécifiquement à la substance de normalisation vers le deuxième emplacement de test ; et
c) l'évaluation des deux premiers signaux détectables au niveau des deux premiers emplacements de test sur la première matrice poreuse,
i) le deuxième signal détectable au niveau du deuxième emplacement de test sur la deuxième matrice poreuse étant évalué et comparé aux deux premiers signaux détectables pour évaluer des taux de 17-bêta estradiol et de progestérone dans ledit échantillon de salive ; et
ii) les taux de 17-bêta estradiol et de progestérone dans l'échantillon de salive étant combinés en un unique résultat de test.

9. Procédé selon la revendication 8, les fragments de Fab marqués, séchés étant solubilisés ou remis en suspension, et transportés vers les deux emplacements de test sur la première matrice poreuse par l'échantillon de salive.

10. Procédé selon l'une quelconque des revendications 8 et 9, qui est utilisé pour évaluer une ovulation, une grossesse, la fertilité, l'état de fertilité, la fenêtre temporelle, une tendance, un suivi ou un guidage de thérapie.

11. Procédé selon la revendication 10, qui est utilisé pour prédire une ovulation, utilisé pour confirmer une grossesse, ou utilisé pour évaluer la fertilité globale et/ou la capacité à concevoir, pour le planning familial et/ou pour le contrôle des naissances.
